(19) Europäisches Patentamt
European Patent Office
Office européen des brevets

(11) Numéro de publication : **0 530 887 A1**

(12) # DEMANDE DE BREVET EUROPEEN

(21) Numéro de dépôt : **92202500.2**

(22) Date de dépôt : **01.08.92**

(51) Int. Cl.[5] : **C07D 493/04**, C07D 277/06, C07K 5/06, A61K 31/425, A61K 31/34, // (C07D493/04, 307:00, 307:00)

(30) Priorité : **07.08.91 FR 9110039**

(43) Date de publication de la demande : **10.03.93 Bulletin 93/10**

(84) Etats contractants désignés : **PT**

(71) Demandeur : **LABORATOIRES HOECHST S.A.**
**TOUR ROUSSEL HOECHST, 1 Terrasse Bellini**
**F-92800 Puteaux (FR)**

(72) Inventeur : **Nallet, Pierre**
**400 rue du Bacon**
**F-69250 Montanay (FR)**
Inventeur : **Dreux, Jacques**
**36 rue Raulin**
**F-69700 Lyon (FR)**
Inventeur : **Berdeaux, Alain**
**63 rue de la Tombe Issoire**
**F-75014 Paris (FR)**
Inventeur : **Richard, Vincent**
**77 rue Broca**
**F-75013 Paris (FR)**
Inventeur : **Martorana, Piero**
**Kaiser-Friedrich-Promenade 108A**
**W-6380 Bad Homburg (DE)**
Inventeur : **Bohn, Helmut**
**Kranzbergring 11**
**W-6369 Schöneck 1 (DE)**

(74) Mandataire : **Urbach, Hans-Georg, Dr. et al**
**Hanauer Landstrasse 526**
**W-6000 Frankfurt am Main 61 (DE)**

(54) **Nitrates organiques, leurs procédés de préparation et leur utilisation dans le traitement de maladies cardiovasculaires.**

(57) Nitrates organiques, leurs procédés de préparation ainsi que leur utilisation dans le traitement de maladies vasculaires et notamment dans le traitement de l'angor.

Lesdits nitrates répondent à la formule I suivante :

$$R\text{-}CO\text{-}(A)_n\text{-}Y\text{-}B \quad (I)$$

dans laquelle :

**R** représente notamment un reste soufré et un résidu d'aminoacide soufré ; **A** représente notamment un groupe $CH_2$ ou un acide aminé substitué ; **n** est égal à 0 ou 1 ou supérieur à 1 ; **Y** représente un atome d'oxygène ou un groupe NH et **B** représente notamment un reste dianhydro 1,4 :3,6 hexitol mononitrate, un reste itol nitrate, un reste d'inositol.

Lesdits nitrates organiques sont préparés en faisant réagir :

I. soit un thioacide de type R-COOH, dans lequel R a la même signification que ci-dessus avec un dérivé de formule II : $(A)_n$-Y-B, dans laquelle A, Y, B et n ont la même signification que ci-dessus,

II. soit un dérivé de formule III : R-CO-$(A)_n$, dans laquelle R, A et n ont la même signification que ci-dessus, avec un dérivé de formule Y-B, dans laquelle Y et B ont la même signification que ci-dessus, dans un solvant approprié et dans des conditions non épimérisantes.

EP 0 530 887 A1

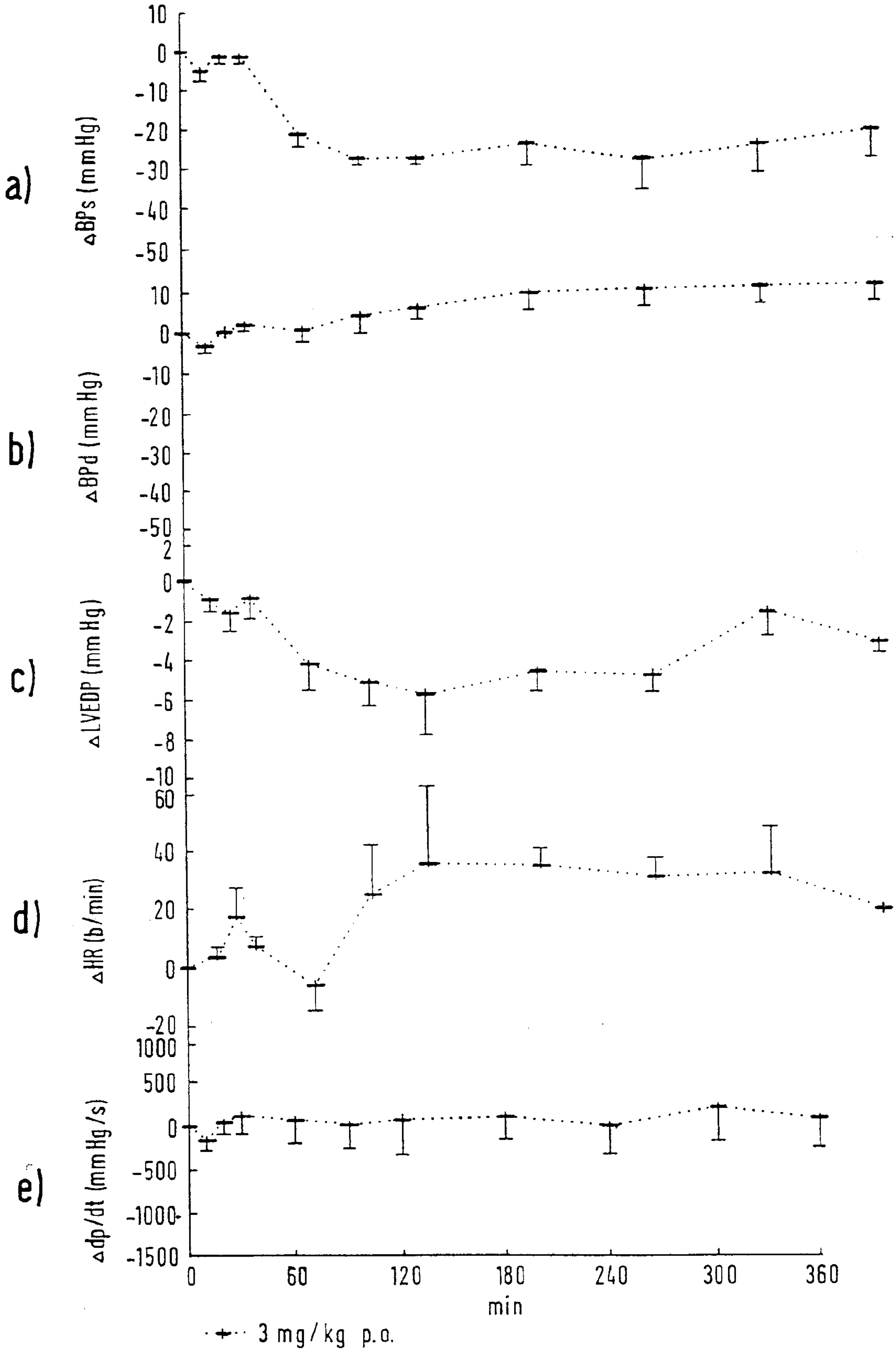
a)
ΔBPs (mmHg)
10
0
-10
-20
-30
-40
-50
b)
ΔBPd (mmHg)
10
0
-10
-20
-30
-40
-50
c)
ΔLVEDP (mmHg)
2
0
-2
-4
-6
-8
-10
d)
ΔHR (b/min)
60
40
20
0
-20
e)
Δdp/dt (mmHg/s)
1000
500
0
-500
-1000
-1500
0
60
120
180
240
300
360
min
3 mg/kg p.o.

Fig. 1

La présente invention est relative à de nouveaux nitrates organiques, à leurs procédés de préparation ainsi qu'à leur utilisation dans le traitement de maladies vasculaires et notamment dans le traitement de l'angor.

La crise d'angine de poitrine résulte d'une ischémie myocardique brutale et réversible et apparaît lorsque les besoins en oxygène du myocarde sont supérieurs aux apports de la circulation coronaire.

Les nitrates organiques, utilisés depuis de nombreuses années dans le traitement de la crise angineuse induisent une relaxation de la fibre musculaire lisse vasculaire en augmentant le taux de la guanylate cyclase soluble, par l'intermédiaire de l'oxyde nitrique formé lors de la transformation des nitrates en présence de cystéine.

De manière générale, leurs effets hémodynamiques sont les suivants :

- vasodilation veineuse, surtout avec diminution du retour veineux, des pressions de remplissage ventriculaire et du volume du ventricule gauche (VG), donc diminution de la tension pariétale ventriculaire ;
- vasodilation artérielle : diminution de la post-charge et diminution du temps d'éjection du ventricule gauche ;
- augmentation réflexe de la fréquence cardiaque et de la contractilité (P. UNGER et al., Rev. Méd. Brux., 1989, 10, 82-88).

Le chef de file de ces produits est la trinitrine, d'action très rapide ; d'autres dérivés ont été développés et présentent une action moins rapide et plus prolongée que cette dernière.

On peut citer, à titre d'exemple de nitrates organiques, outre la trinitrine (nitroglycérine), le tétranitroérythritol, l'hexanitroinositol, le tétranitropentaérythritol, le propatyl-nitrate, le 5-mono-nitrate d'isosorbide (IS-5-MN), le dinitrate d'isosorbide, le 2-mono-nitrate d'isosorbide (IS-2-MN), le 2-nitrate d'isomannide, la trinitrotriéthanolamine, et leurs dérivés substitués, notamment les dérivés aminopropanol des 1,4:3,6-dianhydrohexitol nitrates.

Les nitrates organiques agiraient, après pénétration dans la fibre musculaire lisse, en activant la guanylate cyclase, conformément au schéma ci-après :

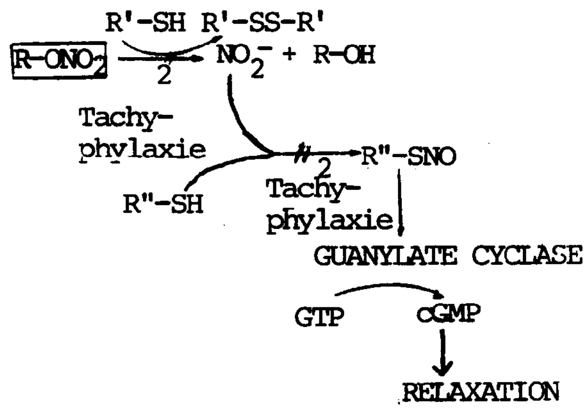


$R\text{-}ONO_2$ = NITROGLYCERINE

qui montre que c'est le S-nitrosothiol (R"-SNO), qui activerait la guanylate cyclase.

La déplétion intracellulaire en groupements thiols serait responsable du phénomène de tachyphylaxie aux nitrates organiques, définie comme la perte d'activité d'une substance lors de son administration répétée, avec la nécessité d'augmenter la dose pour obtenir le même effet.

La prévention de cette tachyphylaxie peut prendre plusieurs formes :

- elle peut consister en l'instauration d'intervalles libres (administrations discontinues, formulations à action brève) (U. ELKAYAM, Ann. Inter. Med., 1991, 114, 8, 667-677) ;
- l'administration simultanée de dérivés de la cystéine ;
- l'administration de dérivés permettant d'éviter cette tachyphylaxie ; la Demande de Brevet EP 362 575 décrit par exemple, des nitrates d'acides gras liés à des acides aminés soufrés, qui empêchent ou atténuent la tachyphylaxie. Cependant, on ne dispose pas, en ce qui concerne les composés décrits dans cette Demande, de recul suffisant tant du point de vue pharmacologique que du point de vue toxicologique.

La Demanderesse s'est en conséquence donné pour but de fournir de nouveaux nitrates organiques qui présentent une activité nettement améliorée (notamment au niveau du transport et du franchissement des barrières cellulaires), ne provoquent aucune tachyphylaxie et dont la toxicologie et la pharmacologie des produits de départ est bien connue.

La présente invention a pour objet des nitrates organiques, caractérisés en ce qu'ils répondent à la formule I suivante :

$$R - CO - (A)_n - Y - B \quad (I)$$

dans laquelle :

<u>R</u> **représente**

a. l'un des restes C, D, E, F et G suivants

- 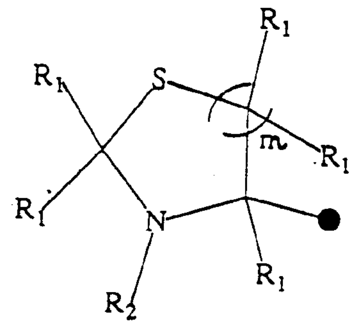 (reste C),

<u>reste C</u> dans lequel :

**$R_1$** représente un atome d'hydrogène, un groupe alkyle en $C_1$ à $C_6$ (linéaire, ramifié ou cyclique), un phényle éventuellement substitué ou un benzyle éventuellement substitué ;

**$R_2$** représente un atome d'hydrogène, un groupe alkyle en $C_1$ à $C_6$ (linéaire, ramifié ou cyclique), un phényle éventuellement substitué, un benzyle éventuellement substitué, un groupe aryle en $C_1$-$C_6$, un benzoyle éventuellement substitué, un alcoxy-carbonyle ou un groupe CO-X dans lequel X représente un reste C ou un reste D, E, F ou G tels que définis ci-après, et **m** représente 1 (cycle à 5 centres) ou 2 (cycle à 6 centres).

- 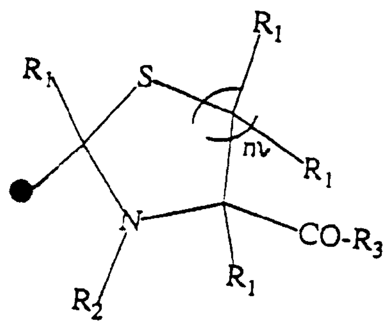 (reste D),

<u>reste D</u> dans lequel :

**$R_1$** et **$R_2$** ont la même signification que ci-dessus ;

**$R_3$** représente un groupe OH, un groupe O-alkyle en $C_1$ à $C_6$ (linéaire, ramifié ou cyclique), un groupe O-phényle éventuellement substitué, un groupe O-benzyle éventuellement substitué, un reste E tel que défini ci-après, un reste de type Y-B- ou un groupe $NH-CH(COOR_3)CH_2-S-R_4$, dans lequel $R_4$ représente un atome d'hydrogène, un groupe alkyle en $C_1$ à $C_6$ (linéaire, ramifié ou cyclique), un phényle éventuellement substitué, un S-phényle éventuellement substitué, un benzyle éventuellement substitué, ou l'un des groupes suivants : $CH_3$-$CO-NH-CH_2$, $B-Y-CO-CH(NH-COO-C(CH_3)_3)CH_2-S$, ou $B-Y-CO-CH(NH_2.HCl)CH_2-S$, B et Y étant tels que définis ci-après, et **m** représente 1 ou 2.

\- 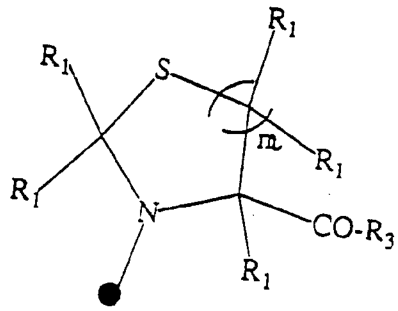 (reste E),

reste E dans lequel :
**$R_1$**, **$R_3$** et **m** ont la même signification que ci-dessus.

\- 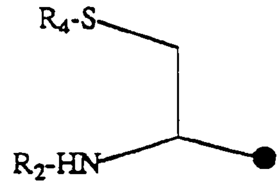 (reste F),

reste F dans lequel :
**$R_2$** et **$R_4$** ont la même signification que ci-dessus.

\- 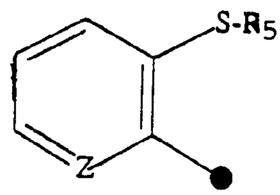 (reste G)

reste G dans lequel :
**$R_5$** représente un atome d'hydrogène, un groupe alkyle en $C_1$ à $C_6$ (linéaire, ramifié ou cyclique), un phényle éventuellement substitué, un S-phényle éventuellement substitué, un benzyle éventuellement substitué, un groupe $CH_3$-CO-NH-$CH_2$ ou B-Y-CO-$C_5ZH_3$-S et **Z** représente CH ou N.

b. un résidu d'aminoacide soufré, éventuellement protégé.

c. R représente également, lorsque n est différent de 0 et A est un reste soufré H ou I tels que définis ci-après, un groupe OH, un groupe O-alkyle en $C_1$-$C_6$ (linéaire, ramifié ou cyclique), un groupe O-phényle éventuellement substitué, un groupe O-benzyle éventuellement substitué, un reste E tel que défini ci-dessus ou un reste Y-B, B et Y étant tels que définis ci-après ;

**A représente** un groupe $CH_2$, un acide aminé substitué ou non, avec la fonction acide liée à Y et la fonction amine liée à CO ou l'un des restes suivants :

\- 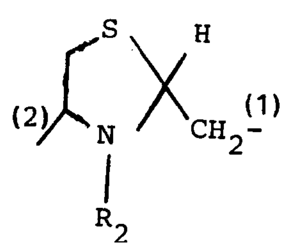 (reste H),

reste H dans lequel :
**$R_2$** représente un atome d'hydrogène, un groupe alkyle en $C_1$ à $C_6$ (linéaire, ramifié ou cyclique), un phényle éventuellement substitué, un benzyle éventuellement substitué, un groupe acyle en $C_1$ à $C_6$, un benzoyle éventuellement substitué, un groupe alcoxy-carbonyle, ou un groupe CO-X dans lequel X représente l'un des restes

C, D, E, F ou G tels que définis ci-dessus pour R,

**(1)** est la liaison avec Y,

**(2)** est la liaison avec R-CO.

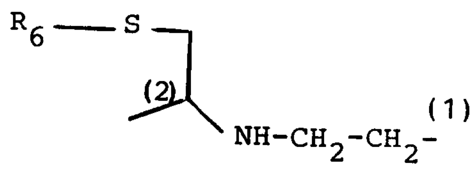

(reste I),

reste I dans lequel :

**$R_6$** représente un atome d'hydrogène, un groupe alkyle en $C_1$ à $C_6$ (linéaire, ramifié ou cyclique), un phényle éventuellement substitué, un S-phényle substitué, un benzyle éventuellement substitué ou l'un des groupes suivants : $CH_3$-CO-NH-$CH_2$, S-$CH_2$-CH-(CO-$R_7$)-NH-$CH_2$-$CH_2$-NH-B dans lequel $R_7$ représente un groupe OH, un groupe O-alkyle en $C_1$-$C_6$, un groupe O-phényle éventuellement substitué, un groupe O-benzyle éventuellement substitué, un reste E tel que défini ci-dessus ou un reste Y-B, B et Y étant tels que définis ci-après,

**(1)** et **(2)** ont la même signification que ci-dessus.

**n est** égal à 0 ou 1 ou supérieur à 1 ;

**Y représente** un atome d'oxygène ou un groupe NH.

**B représente** :

α) un reste dianhydro 1,4:3,6 hexitol mono-nitrate de formule (a)

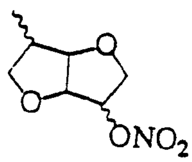

β) un reste itol nitrate en $C_1$ à $C_6$ de formules (b)

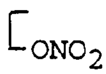 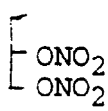 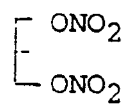 ........→ $C_6$

(b1) (b2) (b3) ....

γ) un reste d'inositol "p" nitrates, p étant un nombre entier de 1 à 5, de formule (c)

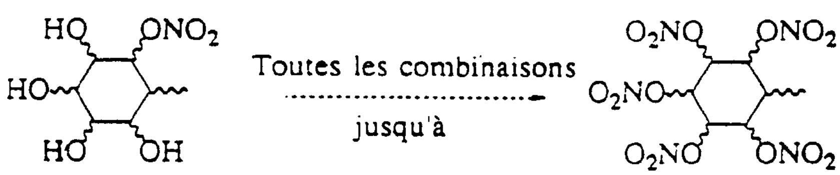


(c1) ............ (c5)

δ) l'un des groupes suivants :

. un groupe $-CH_2-C(CH_2-ONO_2)_3$, dérivé du pentaérythritol,

. un groupe $-CH_2-C(C_2H_5)(CH_2-ONO_2)_2$, dérivé de l'éthyltriméthylol-méthane,

. un groupe $-CH_2-CH_2-N(CH_2-CH_2-ONO_2)_2$, dérivé de la triéthanolamine,

avec toutes les combinaisons OH, $ONO_2$.

Les composés de formule I conformes à l'invention englobent leurs différents isomères.

Dans la présente invention, les substituants des groupes phényle substitué et benzyle substitué, sont avantageusement choisis parmi les groupes suivants : $NO_2$, halogène, $CV_3$ dans lequel V représente un halogène ou un alkyle inférieur.

On obtient ainsi des nitrates organiques qui, de manière inattendue, présentent à la fois une activité nettement améliorée par rapport aux nitrates de l'Art antérieur et n'entraînent pas de tachyphylaxie. De tels nitrates sont particulièrement utiles comme vasorelaxants, notamment dans le traitement de certaines maladies cardiovasculaires et plus particulièrement dans le traitement de l'angine de poitrine.

Ils ont de plus l'avantage de présenter des données pharmacologiques et toxicologiques similaires à celles des nitrates organiques connus qui peuvent, de plus, servir de témoins.

Selon un mode de réalisation avantageux de l'invention, les composés préférés de formule I comprennent pour R, A, Y et B, respectivement les radicaux suivants :

**R représente** l'un des restes suivants :

| |
|---|
| 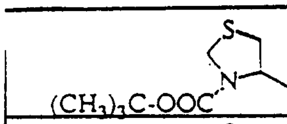 |
| 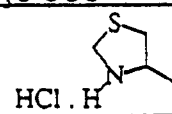 |
| 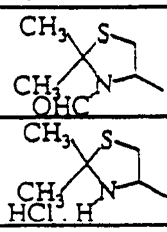 |
| 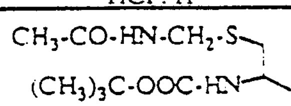 |
| 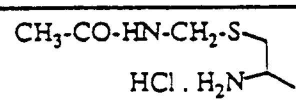 |
| 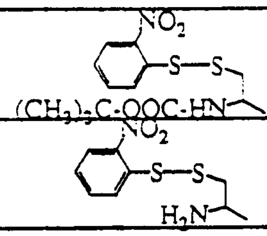 |
| 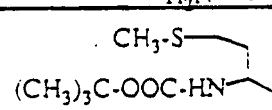 |
| 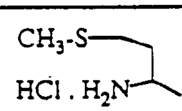 |
| 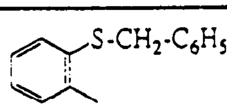 |
| 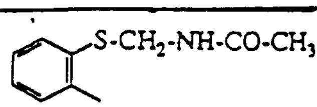 |
| 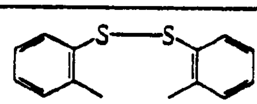 |

**A représente** un reste d'aminoacide éventuellement substitué, lorsque n est différent de 0, et notamment la glycine et ses dérivés, la proline et ses dérivés, l'alanine et ses dérivés, la valine et ses dérivés, et la phénylalanine et ses dérivés.

**Y représente** un atome d'oxygène ou un groupe NH,

**B est** un reste dérivé des composés suivants :

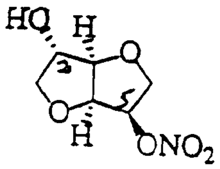

1,4:3,6-dianhydro-D-glucitol-5-nitrate

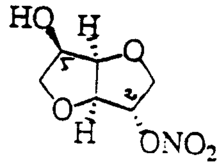

1,4:3,6-dianhydro-D-glucitol-2-nitrate

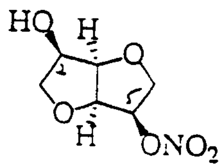

1,4:3,6-dianhydro-D-manitol-5-nitrate

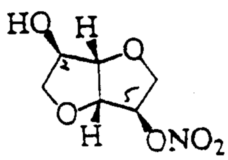

1,4:3,6-dianhydro-D-iditol-5-nitrate

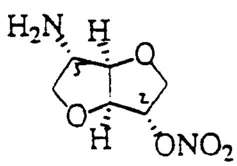

1,4:3,6-dianhydro-5-déoxy-L-iditol-5-amino-2-nitrate

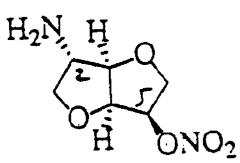

1,4:3,6-dianhydro-2-déoxy-D-glucitol-2-amino-5-nitrate

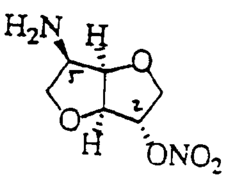

1,4:3,6-dianhydro-5-déoxy-D-glucitol-5-amino-2-nitrate

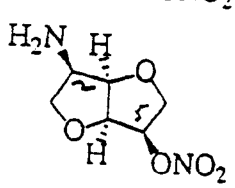

1,4:3,6-dianhydro-2-déoxy-D-manitol-2-amino-5-nitrate.

De manière générale, les nitrates organiques conformes à l'invention comprennent un thioacide R-COOH associé à un nitrate organique de type itol ou dianhydro itol, au niveau d'une fonction OH ou NH dudit nitrate. On distingue ainsi plusieurs restes selon le radical R de l'acide associé au nitrate :

. thioproline et ses dérivés (restes C, D, E) ;
. dérivés de la cystéine (reste F) et des autres acides aminés soufrés ;
. dérivés de l'acide thiosalicylique (reste G) ou de l'acide 3-mercaptopicolinique.

De manière inattendue, l'ensemble de ces produits présentent à la fois une activité sur le système cardiovasculaire ( activité vasorelaxante ), et notamment une activité antiangineuse et une diminution du phénomène de tachyphylaxie.

La présente invention a également pour objet un procédé de préparation d'un nitrate organique conforme à l'invention, caractérisé en ce que l'on fait réagir :

I. soit un thioacide de type R-COOH, dans lequel R a la même signification que ci-dessus avec un dérivé de formule II : $(A)_n$-Y-B, dans laquelle A, Y, B et n ont la même signification que ci-dessus,

II. soit un dérivé de formule III : R-CO-$(A)_n$, dans laquelle R, A et n ont la même signification que ci-dessus, avec un dérivé de formule Y-B, dans laquelle Y et B ont la même signification que ci-dessus, dans un solvant approprié et dans des conditions non épimérisantes.

De manière avantageuse, le solvant est notamment et ce de manière non limitative : du dichlorométhane, de l'acétonitrile, du diméthylformamide, de l'acétate d'éthyle ou du tétrahydrofuranne.

Selon un mode de mise en oeuvre avantageux dudit procédé, les dérivés de formule II sont choisis parmi :

| | |
|---|---|
| A | $C_6H_5-H_2C-OOC-HN-CH_2-CO$ |
| | $(CH_3)_3C-OOC-HN-CH_2-CO$ |
| | $HCl . H_2N-CH_2-CO$ |
| | $(CH_3)_3C-OOC-HN-CH-CO$<br>$CH_3$ |
| | $HCl . H_2N-CH-CO$<br>$CH_3$ |
| | $(CH_3)_3C-OOC-HN-CH-CO$<br>$(CH_3)_2CH$ |
| | $HCl . H_2N-CH-CO$<br>$(CH_3)_2CH$ |
| | $(CH_3)_3C-OOC-HN-CH-CO$<br>$C_6H_5-CH_2$ |
| | $HCl . H_2N-CH-CO$<br>$C_6H_5-CH_2$ |
| | 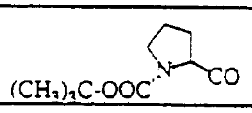 |
| | 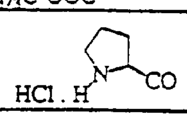 |

| Y-B | | | |
|---|---|---|---|
| 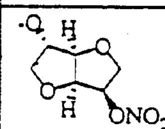 | 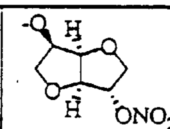 | 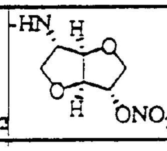 | 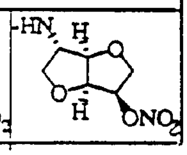 |

Selon un autre mode de mise en oeuvre avantageux dudit procédé, le dérivé de formule III est :

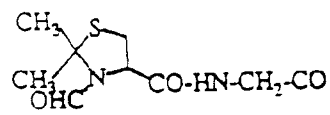

De manière inattendue, les dérivés de formule II présentent une activité sur le système cardiovasculaire et notamment une activité antigineuse.

Aussi bien les dérivés de formule I que les dérivés de formule II trouvent application comme médicaments dans le traitement de maladies cardiovasculaires et notamment dans le traitement de l'angine de poitrine.

Outre les dispositions qui précèdent l'invention comprend encore d'autres dispositions, qui ressortiront de la description qui va suivre, qui se réfère à des exemples de mise en oeuvre du procédé objet de la présente invention.

Il doit être bien entendu, toutefois, que ces exemples sont donnés uniquement à titre d'illustration de l'objet de l'invention, dont ils ne constituent en aucune manière une limitation.

**EXEMPLES :**

**I - SYNTHESE DES INTERMEDIAIRES.**

**I-a : de type $(A)_n$-Y-B de formule II, avec n = 1 :**

Le Tableau I ci-après montre les différents dérivés synthétisés.

TABLEAU I

| | | Y-B | | | |
|---|---|---|---|---|---|
| | Nitrates organiques / Acides aminés | 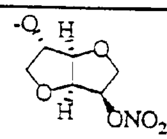 | 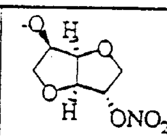 | 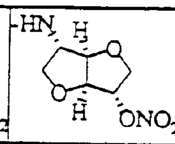 | 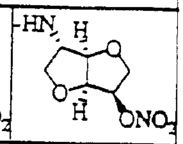 |
| A | $C_6H_5$-$H_2C$-OOC-HN-$CH_2$-CO | | | 4 | |
| | $(CH_3)_3C$-OOC-HN-$CH_2$-CO | 1 | | 5 | 15 |
| | HCl . $H_2N$-$CH_2$-CO | 2 | 3 | 6 | 16 |
| | $(CH_3)_3C$-OOC-HN-CH-CO<br>$CH_3$ | | | 7 | |
| | HCl . $H_2N$-CH-CO<br>$CH_3$ | | | 8 | |
| | $(CH_3)_3C$-OOC-HN-CH-CO<br>$(CH_3)_2CH$ | | | 9 | |
| | HCl . $H_2N$-CH-CO<br>$(CH_3)_2CH$ | | | 10 | |
| | $(CH_3)_3C$-OOC-HN-CH-CO<br>$C_6H_5$-$CH_2$ | | | 11 | |
| | HCl . $H_2N$-CH-CO<br>$C_6H_5$-$CH_2$ | | | 12 | |
| | $(CH_3)_3C$-OOC 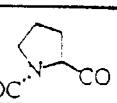 | | | 13 | |
| | HCl . H 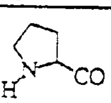 | | | 14 | |

Dans les exemples qui suivent, les abrévations utilisées ont les significations ci-après :

| | | |
|---|---|---|
| Boc | pour | butoxycarbonyl |
| DCC | " | 1,3-dicyclohexylcarbodiimide |
| DCU | " | 1,3-dicyclohexylurée |
| HOBt | " | 1-hydroxybenzotriazole-hydrate |
| CMC | " | 1-cyclohexyl-3-(2-morpholinoéthyl)carbodiimide métho-p-toluènesulfonate |
| CMU | " | 1-cyclohexyl-3-(2-morpholinoéthyl)urée métho-p-toluènesulfonate |
| CBZ | " | benzyloxycarbonyl. |

Exemple 1 : **Glycine, N-[(t-butoxy)carbonyl]-, ester du 5-O-nitro, 1,4:3,6-dianhydro-D-glucitol (1) :**

1 g (5,23 mmoles) de 1,4:3,6-dianhydro-D-glucitol-5-nitrate, 0,92 g (5,25 mmoles) de N-t-Boc glycine, 2,22 g (5,24 mmoles) de CMC et 0,08 g (0,52 mmoles) de 4-pyrrolidinopyridine sont agités 24 heures à température ambiante dans 40 ml de dichlorométhane (stabilisé sur amylène et séché sur alumine). Après filtration du CMU, on ajoute 50 ml de dichlorométhane et la solution est lavée successivement avec 30 ml d'une solution à 5 % d'acide acétique, 30 ml d'eau, 30 ml d'une solution demi-saturée en bicarbonate de sodium, trois fois 30 ml d'eau, puis séchée sur sulfate de sodium. Après filtration et évaporation, le produit est recristallisé dans l'acétate d'éthyle. On obtient 0,85 g (Rdt = 47 %).
F = 130-131°C.

| Analyse | C | H | N |
|---|---|---|---|
| Calc. (0,45 $H_2O$) | 43,80 | 5,90 | 7,85 |
| Tr. | 43,9 | 5,7 | 7,7 |

Exemple 2 : **Glycine, ester du 5-O-nitro, 1,4:3,6-dianhydro-D-glucitol, monochlorhydrate (2) :**

A 45 ml d'une solution d'acide chlorhydrique 2 N dans l'acétate d'éthyle, on ajoute 6,6 g du produit précédent (1). La solution est agitée 10 heures à température ambiante et le précipité formé est filtré et lavé à l'éther puis recristallisé dans le méthanol. On obtient 3,2 g (Rdt = 59 %) de produit purifié.
F = 196°C (déc), $[\alpha]_D^{20}$= +113,5 (c 0,6 eau).

| Analyse | C | H | N | O |
|---|---|---|---|---|
| Calc. (0,16 $H_2O$) | 33,40 | 4,66 | 9,73 | 39,81 |
| Tr. | 33,4 | 4,6 | 9,6 | 40,1 |

Exemple 3 : **Glycine, ester du 2-O-nitro, 1,4:3,6-dianhydro-D-glucitol, monochlorhydrate (3) :**

4 g (20,9 mmoles) de 1,4:3,6-dianhydro-D-glucitol-2-nitrate, 3,4 g (19,4 mmoles) de N-t-Boc glycine, 8,8 g (20,9 mmoles) de CMC et 0,28 g (2 mmoles) de 4-pyrrolidinopyridine sont agités 24 heures à température ambiante dans 120 ml de dichlorométhane (stabilisé sur amylène et séché sur alumine). Après filtration du CMU, on ajoute 130 ml de dichlorométhane et la solution est lavée et séchée comme pour (1). On isole 5,85 g d'une huile correspondant au produit attendu. Cette huile est solubilisée dans 42 ml d'une solution d'acide chlorhydrique 2 N dans l'acétate d'éthyle. Après 10 heures à température ambiante, le précipité formé est filtré et lavé à l'éther puis recristallisé dans le méthanol anhydre. On obtient 2,5 g (Rdt = 45 %).
F = 188-189°C (déc), $[\alpha]_D^{20}$= +88,1 (c 0,6 eau).

| Analyse | C | H | N |
|---|---|---|---|
| Calc. | 33,75 | 4,60 | 9,84 |
| Tr. | 34,0 | 4,6 | 9,8 |

Exemple 4 : **Glycine, N-[(benzyloxy)carbonyl]-, amide du 5-amino, 5-déoxy, 2-O-nitro, 1,4:3,6-dianhydro-L-iditol (4) :**

On agite à température ambiante une solution de 1,43 g (7,5 mmoles) de 1,4:3,6-dianhydro-5-déoxy-L-iditol-5-amino-2-nitrate dans 70 ml de dichlorométhane anhydre. On ajoute successivement 1,54 g (7,41 mmoles) de N-CBZ glycine et 1, 53 g (7,41 mmoles) de DCC. Après 24 heures de réaction, filtration du DCU et évaporation du solvant, on chromatographie l'huile obtenue ( silice 230-400 mesh ASTM). Le composé isolé est cristallisé avec de l'éther puis recristallisé dans le mélange acétate d'éthyle-éther diisopropylique. On obtient 2 g (Rdt = 70 %).
F = 75-76°C, $[\alpha]_D^{20}$= +24,7 (c 1, éthanol).

| Analyse | C | H | N |
|---|---|---|---|
| Calc. | 50,39 | 5,02 | 11,01 |
| Tr. | 50,2 | 5,1 | 10,9 |

Exemple 5 : **Glycine, N-[(t-butoxy)carbonyl]-, amide du 5-amino, 5-déoxy, 2-O-nitro, 1,4:3,6-dianhydro-L-iditol (5) :**

On agite à température ambiante une solution de 6 g (31,5 mmoles) de 1,4:3,6-dianhydro-5-déoxy-L-iditol-5-amino-2-nitrate dans 270 ml de dichlorométhane anhydre. On ajoute successivement 5,4 g (30,8 mmoles) de N-t-Boc glycine et 6,4 g (31 mmoles) de DCC. Après 24 heures de réaction, filtration du DCU et évaporation du solvant, on chromatographie sur silice (230-400 mesh ASTM) et on recristallise dans le méthanol le composé isolé. On obtient 7,75 g (Rdt = 72 %).
F = 118°C.

Exemple 6 : **Glycine, amide du 5-amino, 5-déoxy, 2-O-nitro, 1,4:3,6-dihydro-L-iditol, monochlorhydrate (6) :**

A 35 ml d'une solution d'acide chlorhydrique 1,7 N dans le méthanol, on ajoute 4,75 g du composé précédent (5). La solution est agitée 10 heures à température ambiante et le précipité formé est filtré et lavé à l'éther anhydre. On recristallise dans le méthanol, on obtient 3,3 g (Rdt = 85 %).
F = 198°C (déc), $[\alpha]_D^{20}$= -2,5 (c 1, eau).

| Analyse | C | H | N | Cl |
|---|---|---|---|---|
| Calc. | 33,87 | 4,97 | 14,81 | 12,49 |
| Tr. | 34,1 | 4,8 | 14,7 | 12,3 |

La forme base de ce composé est préparée ainsi : dans une suspension de 2,35 g de (6) or fait barboter de l'ammoniac à température ambiante et sous agitation. On filtre le chlorure d'ammonium formé et après évaporation du solvant, on observe une cristallisation de l'huile résiduelle. Après recristallisation dans le mélange acétate d'éthyle-éther diisopropylique, on obtient 1,85 g (Rdt = 90 %).
F = 81°C.

Exemple 7 : **L-alanine, N-[(t-butoxy)carbonyl]-, amide du 5-amino, 5-déoxy, 2-O-nitro, 1,4:3,6-dianhydro-L-iditol (7) :**

On agite à température ambiante une solution de 2 g (10,5 mmoles) de 1,4:3,6-dianhydro-5-déoxy-L-iditol-5-amino-2-nitrate dans 70 ml de dichlorométhane anhydre. On ajoute successivement 2 g (10,5 mmoles) de N-t-Boc-L-alanine, 4,46 g (10,5 mmoles) de CMC et 1,42 g (10,5 mmoles) d'HOBt. Après 24 heures de réaction, on ajoute du dichlorométhane et la phase organique est lavée et séchée comme pour (1). On isole 3,15 g d'un solide qui est recristallisé dans le méthanol. On obtient 2,15 g (Rdt = 56 %).
F = 133°C, $[\alpha]_D^{20}$= +15,8 (c 0,6 éthanol).

| Analyse | C | H | N |
|---|---|---|---|
| Calc. | 46,53 | 6,41 | 11,62 |
| Tr. | 46,8 | 6,4 | 11,5 |

Exemple 8 : **L-alanine, amide du 5-amino, 5-déoxy, 2-O-nitro, 1,4:3,6-dihydro-L-iditol monochlorohydrate (8) :**

A 38 ml d'une solution d'acide chlorhydrique 1,7 N dans le méthanol, on ajoute 3,05 g du composé précédent (7). La suspension est agitée 24 heures à température ambiante (le milieu devient limpide après 3 heures de réaction), puis on concentre sous vide et on précipite un solide blanc par ajout d'éther. On obtient 2,6 g de solide que l'on recristallise dans le méthanol. On récupère finalement 2,1 g (Rdt = 83 %).
F = 189°C (déc), $[\alpha]_D^{20}$= +9,3 (c 0,6 eau).

| Analyse | C | H | N | Cl |
|---|---|---|---|---|
| Calc. | 36,31 | 5,42 | 14,11 | 11,90 |
| Tr. | 36,5 | 5,5 | 14,2 | 11,9 |

Exemple 9 : **L-valine, N-[(t-butoxy)carbonyl]-, amide du 5-amino, 5-déoxy, 2-O-nitro, 1,4:3,6-dianhydro-L-iditol (9) :**

On agite à température ambiante une solution de 3,4 g (17,8 mmoles) de 1,4:3,6-dianhydro-5-déoxy-L-iditol-5-amino-2-nitrate dans 220 ml de dichlorométhane anhydre. On ajoute successivement 3,7 g (17 mmoles) de N-t-Boc-L-valine, 7,56 g (17,8 mmoles) de CMC et 2,4 g (17,8 mmoles) d'HOBt. Après 21 heures de réaction, on ajoute du dichlorométhane et on procède comme pour la préparation de (1) ; le solide est alors recristallisé dans le mélange méthanol-éther de pétrole ; on obtient 3,55 g (Rdt = 54 %).
F = 130-131°C, $[\alpha]_D^{20}$= +30,66 (c 0,6 éthanol).

| Analyse | C | H | N |
|---|---|---|---|
| Calc. | 49,35 | 6,98 | 10,79 |
| Tr. | 49,4 | 6,7 | 10,6 |

Le traitement de ce composé par une solution d'acide chlorhydrique 2N dans l'acétate d'éthyle permet d'enlever le groupement t-butoxy-carbonyl et conduit au chlorhydrate d'amine correspondant (10). Ce composé n'a pas été recristallisé.

Exemple 10 : **L-phénylalanine, N-[(t-butoxy)carbonyl]-, amide du 5-amino, 5-déoxy, 2-O-nitro, 1,4:3,6-dianhydro-L-iditol (11) :**

On agite à température ambiante une solution de 3,8 g (20 mmoles) de 1,4:3,6-dianhydro-5-déoxy-L-iditol-5-amino-2-nitrate dans 250 ml de dichlorométhane anhydre. On ajoute successivement 5,2 g (19,6 mmoles) de N-t-Boc-L-phénylalanine, 8,48 g (20 mmoles) de CMC et 2,7 g (20 mmoles) d'HOBt. Après 24 heures de réaction, on procède comme pour la préparation (1) ; le solide est alors recristallisé dans l'éthanol absolu ; on obtient 4,45 g (Rdt = 52 %) d'un solide blanc.
F = 157-159°C, $[\alpha]_D^{20}$= +25 (c 0,6 éthanol).

| Analyse | C | H | N |
|---|---|---|---|
| Calc. | 54,91 | 6,22 | 9,60 |
| Tr. | 54,7 | 6,2 | 9,6 |

Exemple 11 : **L-phénylalanine, amide du 5-amino, 5-déoxy, 2-O-nitro, 1,4:3,6-dianhydro-L-iditol, monochlorhydrate (12) :**

A 50 ml d'une solution d'acide chlorhydrique 2 N dans le méthanol, on ajoute 4,2 g du composé précédent (11). On agite 20 heures à température ambiante puis on concentre sous vide, la masse visqueuse obtenue est chromatographiée sur silice (230-400 mesh ASTM). On isole une huile qui est traitée par de l'éthanol ; on élimine la fraction huileuse insoluble et on précipite un solide blanc de la phase éthanolique par ajout d'éther. Le produit est recristallisé dans le mélange méthanol-éther diisopropylique. On obtient 2,15 g de produit, (Rdt = 60 %).
F = 175°C (déc), $[\alpha]_D^{20}$= +53 (c 0,6 eau)

| Analyse | C | H | N |
|---|---|---|---|
| Calc. (0,7 $H_2O$) | 46,62 | 5,57 | 10,87 |
| Tr. | 46,5 | 5,4 | 10,9 |

Exemple 12 : **L-proline, N-[(t-butoxy)carbonyl]-, amide du 5-amino, 5-déoxy, 2-O-nitro, 1,4:3,6-dianhydro-L-iditol (13) :**

On agite à température ambiante une solution de 2,5 g (13,1 mmoles) de 1,4:3,6-dianhydro-5-déoxy-L-iditol-5-amino-2-nitrate dans 125 ml de dichlorométhane anhydre. On ajoute successivement 2,8 g (13,0 mmoles) de N-t-Boc-L-proline, 5,5 g (13 mmoles) de CMC et 1,75 g (13 mmoles) d'HOBt. Après 24 heures de réaction, on procède comme pour la préparation de (1) ; le solide obtenu est recristallisé dans l'acétate d'éthyle, on obtient 2,9 g (Rdt = 57 %).
F = 117-118°C, $[\alpha]_D^{20}$= +5,2 (c 0,6 éthanol).

| Analyse | C | H | N |
|---|---|---|---|
| Calc. | 49,60 | 6,50 | 10,84 |
| Tr. | 49,7 | 6,7 | 10,6 |

Exemple 13 : **L-proline, amide du 5-amino, 5-déoxy, 2-O-nitro, 1,4:3,6-dianhydro-L-iditol monochlorohydrate (14) :**

A 17 ml d'une solution d'acide chlorhydrique 2,2 N dans le méthanol, on ajoute 3,3 g de composé précédent (13). On agite 20 heures à température ambiante puis on concentre sous vide et on précipite le produit par ajout d'éther. Après recristallisation dans l'éthanol, on obtient 2,2 g (Rdt = 80 %) d'un solide blanc.
F = 173°C (déc), $[\alpha]_D^{20}$= -28,1 (c 0,6 eau).

| Analyse | C | H | N | Cl |
|---|---|---|---|---|
| Calc. | 40,81 | 5,60 | 12,97 | 10,95 |
| Tr. | 40,7 | 5,8 | 12,8 | 11,1 |

Exemple 14 : **Glycine, N-[(t-butoxy)carbonyl]-, amide du 2-amino, 2-déoxy, 5-O-nitro, 1,4:3,6-dianhydro-D-glucitol (15) :**

On agite à température ambiante une solution de 4 g (21 mmoles) de 1,4:3,6-dianhydro-2-déoxy-D-glucitol-2-amino-5-nitrate dans 180 ml de dichlorométhane anhydre. On ajoute successivement 3,6 g (20,5 mmoles) de N-t-Boc glycine et 4,28 g (20,7 mmoles) de DCC. Après 24 heures de réaction, filtration du DCU et évaporation du solvant, on chromatographie sur silice (230-400 mesh ASTM). Le composé isolé est recristallisé dans le mélange acétate d'éthyle-éther diisopropylique ; on obtient 4,8 g (Rdt = 67 %).
F = 112-113°C, $[\alpha]_D^{20}$= +101,8 (c 0,6 éthanol).

| Analyse | C | H | N |
|---|---|---|---|
| Calc. | 44,95 | 6,09 | 12,10 |
| Tr. | 45,0 | 6,2 | 12,0 |

Exemple 15 : **Glycine, amide du 2-amino, 2-déoxy, 5-O-nitro, 1,4:3,6-dianhydro-D-glucitol monochlorhydrate (16) :**

A 60 ml d'une solution d'acide chlorhydrique 1,85 N dans le méthanol, on ajoute 7,3 g du composé précédent (15). On agite 16 heures à température ambiante, on filtre le précipité formé et on le lave à l'éther anhydre. On recristallise ce composé dans le méthanol et on obtient 2,95 g (Rdt = 49 %) de solide blanc.
F = 193-194°C, $[\alpha]_D^{20}$= +95 (c 0,6 eau).

| Analyse | C | H | N | Cl |
|---|---|---|---|---|
| Calc. | 33,87 | 4,87 | 14,81 | 12,49 |
| Tr. | 33,7 | 5,1 | 14,6 | 12,5 |

**I-b : de type $(A)_n$-Y-B avec n = 2 :**

Exemple 16 : **5-[[2-[[(2-pyrrolidinyl (2S)) carbonyl] amino]-1-oxo éthyl] amino]-, 5-déoxy, 2-O-nitro, 1,4:3,6-dianhydro-L-iditol monochlorhydrate (49) :**

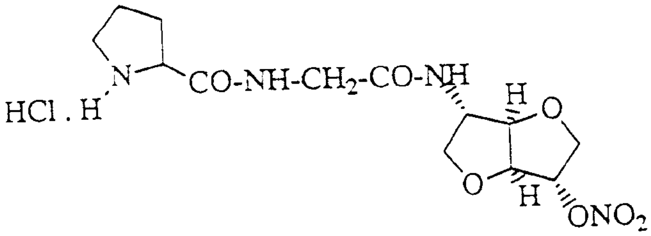

(49)

On agite à température ambiante une solution de 5,35 g (21,6 mmoles) de la forme base correspondant à (6) dans 190 ml de dichlorométhane anhydre. On ajoute successivement 4,65 g (21,6 mmoles) de N-t-Boc-L-proline, 4,46 g (21,6 mmoles) de DCC et 2,92 g (21,6 mmoles) d'HOBt. Après 24 heures de réaction, on filtre le DCU et on évapore le solvant. On chromatographie l'huile sur silice (230-400 mesh ASTM) et on isole 7,8 g (Rdt = 81 %) du composé attendu. Celui ci n'a pu être cristallisé ; il est traité directement.

A 60 ml d'une solution d'acide chlorhydrique 2N dans l'acétate d'éthyle, on ajoute les 7,8 g du composé précédent. La solution est agitée 6 heures à température ambiante et le solide blanc formé est filtré, lavé à l'acétate d'éthyle puis à l'éther. Après recristallisation dans l'éthanol, puis dans le méthanol, on obtient 4,5 g de produit (Rdt = 67 %).
F = 179°C (déc), $[\alpha]_D^{20}$= -21,1 (c 1, eau).

| Analyse | C | H | N | Cl |
|---|---|---|---|---|
| Calc. (0,04 HCl) | 40,85 | 5,50 | 14,65 | 9,64 |
| Tr. | 40,7 | 5,5 | 14,6 | 9,6 |

**I-c : de type R-CO-A de formule III :**

Exemple 17 : **N-[4-(3-formyl-2,2-diméthyl-L-thiazolidine) carbonyl]-, glycine (17).**

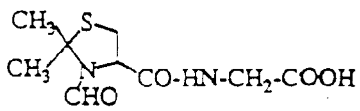

(17)

Ce composé est préparé par saponification de l'ester correspondant.

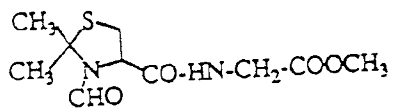

dont la synthèse est décrite par KING et al., J. Chem. Soc. 1957, 880-885.

On agite à température ambiante une solution de 6,24 g d'ester dans 63 ml de dioxanne et on ajoute 25,8 ml d'une solution 1 N de soude. Après une heure de réaction, on concentre la solution et on acidifie avec 25 ml d'une solution 1 N d'acide chlorhydrique. Le précipité blanc est filtré, lavé avec du tétrachlorure de carbone puis à l'éther. On obtient 4,35 g d'acide (Rdt = 74 %) de pureté suffisante pour être engagé dans l'étape suivante.

## II - SYNTHESE DES COMPOSES DE FORMULE I avec n = 0.

Le Tableau II ci-après montre les différents dérivés synthétisés.

TABLEAU II

| | Y-B | | | |
|---|---|---|---|---|
| R / Nitrates organiques | 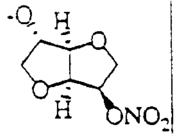 | 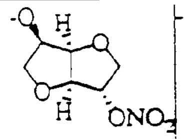 | 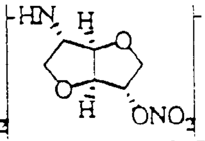 | 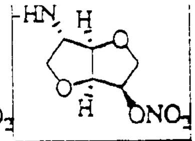 |
| 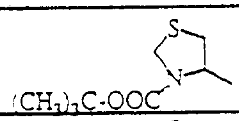 | 18 | 24 | 50 | |
| 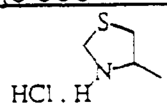 | 19 | 25 | 30 | 40 |
| 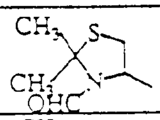 | | | 31 | |
| 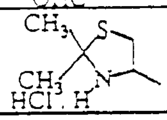 | | | 32 | |
| 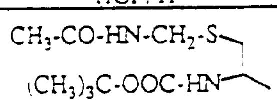 | 20 | 26 | 33 | 41 |
| 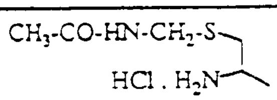 | 21 | 27 | 34 | 42 |
| 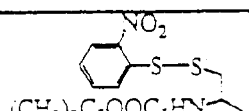 | | | 35 | |
| 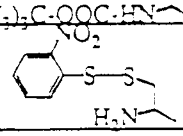 | | | 36 | |
| 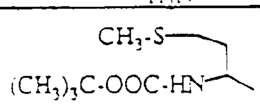 | | | 37 | 43 |
| 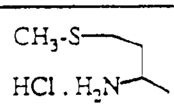 | | | 38 | 44 |
| 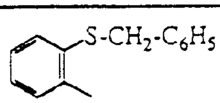 | 22 | 28 | 39 | 45 |
| 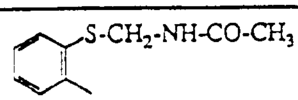 | 23 | | | |
| 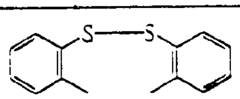 | | 29 | | |

| Nitrates organiques / R | Y-B | | | |
|---|---|---|---|---|
| | 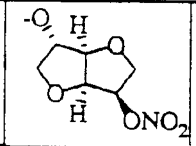 | 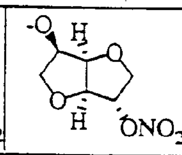 | 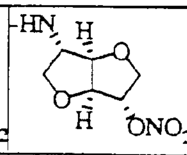 | 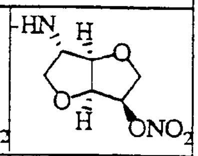 |
| 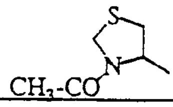 | | | 51 | |
| 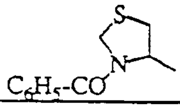 | | | 52 | |
| 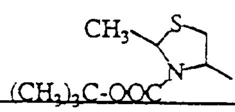 | | | 53 | |
| 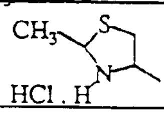 | | | 54 | |
| 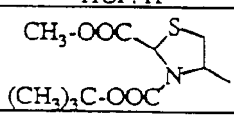 | | | 55 | |
| 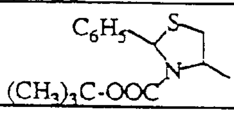 | | | 56 | |
| 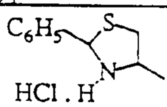 | | | 57 | |
| 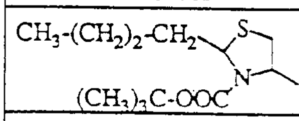 | | | 58 | |
| 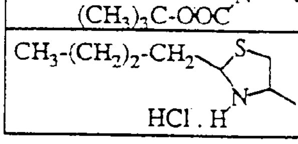 | | | 59 | |

Exemple 18 : **2-O-[(3-t-butoxycarbonyl-4-L-thiazolidinyl)carbonyl]-, 5-O-nitro, 1,4:3,6-dianhydro-D-glucitol (18) :**

5 g (26,1 mmoles) de 1,4:3,6-dianhydro-D-glucitol-5-nitrate, 6,15 g (26,3 mmoles) de N-t-Boc-L-thioproline, 5,4 g (26,1 mmoles) de DCC, 3,55 g (26,2 mmoles) d'HOBt et 0,435 g (2,9 mmoles) de 4-pyrrolidinopyridine sont agités dans 225 ml de dichlorométhane (stabilisé sur amylène et séché sur alumine) pendant 24 heures à température ambiante. Après filtration du DCU, on procède comme pour (1). Le résidu d'évaporation est chromatographié sur silice (230-400 mesh ASTM) et le produit isolé est recristallisé dans l'acétate d'éthyle puis lavé à l'éther de pétrole. On obtient 5,5 g (Rdt = 51 %).
F = 106-110°C, $[\alpha]_D^{20}$= +10,6 (c 0,6 acétone).

| Analyse | C | H | N |
|---|---|---|---|
| Calc. | 44,33 | 5,45 | 6,89 |
| Tr. | 44,4 | 5,5 | 6,8 |

Exemple 19 : **2-O-[(4-L-thiazolidinyl)carbonyl]-, 5-O-nitro, 1,4:3,6-dianhydro-D-glucitol, monochlorhydrate (19) :**

A 32 ml d'une solution d'acide chlorhydrique 2 N dans l'acétate d'éthyle, on ajoute 2,5 g (6,1 mmoles) du produit précédent (18). La solution est agitée 1 heure à température ambiante et le précipité formé est filtré puis lavé à l'éther. Après recristallisation dans l'éthanol, on obtient 2,85 g de produit (Rdt = 76 %).
F = 183°C (déc), $[\alpha]_D^{20}$= +42,8 (c 0,6 eau).

| Analyse | C | H | N |
|---|---|---|---|
| Calc. | 35,04 | 4,41 | 8,17 |
| Tr. | 34,7 | 4,4 | 8,1 |

Exemple 20 : **L-cystéine, S-[(acétylamino)méthyl]-N-[(t-butoxy)carbonyl]-, ester du 5-O-nitro, 1,4:3,6-dianhydro-D-glucitol (20) :**

1,91 g (10 mmoles) de 1,4:3,6-dianhydro-D-glucitol-5-nitrate, 3,5 g (12 mmoles) de N-t-Boc-S-acétamidométhyl-L-cystéine (12 mmoles), 2,47 g (12 mmoles) de DCC, 1,6 g (12 mmoles) d'HOBt et 0,2 g (1,3 mmoles) de 4-pyrrolidinopyridine sont agités dans 100 ml de dichlorométhane (stabilisé sur amylène et séché sur alumine) pendant 6 heures. Après filtration du DCU, la solution est lavée et séchée comme pour (1). Le produit isolé après chromatographie est recristallisé dans le mélange acétone-éther diisopropylique (50-50). On obtient 3,4 g (Rdt = 73 %).
F = 105-106°C, $[\alpha]_D^{20}$= +59 (c 0,5 acétone).

| Analyse | C | H | N | O |
|---|---|---|---|---|
| Calc. | 43,87 | 5,85 | 9,03 | 34,37 |
| Tr. | 43,8 | 5,8 | 8,7 | 34,1 |

Exemple 21 : **L-cystéine, S-[(acétylamino)méthyl]-, ester du 5-O-nitro, 1,4:3,6-dianhydro-D-glucitol, monochlorhydrate (21) :**

A 24 ml d'une solution d'acide chlorhydrique 2 N dans l'acétate d'éthyle, on ajoute 2,9 g (6,2 mmoles) du produit précédent (20). La solution est agitée 5 heures à température ambiante et le précipité formé est filtré puis lavé à l'éther. Après chromatographie sur silice (230-400 mesh ASTM), le produit est recristallisé du mélange éthanol-acétone. On obtient 2,15 g (Rdt = 85 %).
F = 152-155°C.

Exemple 22 : **2-O-[2-[(phénylméthyl)thio]benzoyl]-, 5-O-nitro, 1,4:3,6-dianhydro-D-glucitol (22) :**

10,5 g (40 mmoles) de chlorure d'acide thiosalicylique S-benzylé sont dissous dans 100 ml de pyridine anhydre et introduits dans le réacteur. On coule alors goutte à goutte à température ambiante une solution de 5,7 g (30 mmoles) de 1,4:3,6-dianhydro-D-glucitol-5-nitrate dans 50 ml de pyridine anhydre. Sous agitation, on chauffe à 40°C le mélange réactionnel pendant 24 heures. On évapore la pyridine sans dépasser 40°C et le résidu est repris dans le dichlorométhane, lavé à l'eau, à l'acide chlorhydrique 1 N, à l'eau, avec une solution aqueuse de bicarbonate de sodium, puis à l'eau jusqu'à neutralité et séché sur sulfate de sodium. Le produit visqueux est alors chromatographié sur silice (230-400 mesch ASTM) et le composé isolé est recristallisé dans le mélange éther diisopropylique-acétate d'éthyle 80-20. On obtient 6,1 g de produit (Rdt = 49 %).
F = 98-99°C.

| Analyse | C | H | N |
|---|---|---|---|
| Calc. | 57,55 | 4,59 | 3,35 |
| Tr. | 57,4 | 4,4 | 3,3 |

Exemple 23 : **2-O-[2-[(acétylamino)méthyl]thio]benzoyl]-, 5-O-nitro, 1,4:3,6-dianhydro-D-glucitol (23) :**

3,8 g (20 mmoles) de 1,4:3,6-dianhydro-D-glucitol-5-nitrate, 5 g (22 mmoles) d'acide S-acétaminométhyle thiosalicyclique, 4,5 g (22 mmoles) de DCC et 0,44 g (3 mmoles) de 4-pyrrolidinopyridine sont agités 48 heures à température ambiante dans 60 ml de dichlorométhane (stabilisé sur amylène, séché sur alumine). Après filtration du DCU, la solution est lavée et séchée comme pour (1). Le produit isolé après chromatographie est recristallisé dans l'éthanol. On obtient 5,6 g (Rdt = 67 %).
F = 126-127°C, $[\alpha]_D^{20}$= +93,4 (c 0,5 acétone).

| Analyse | C | H | N | O |
|---|---|---|---|---|
| Calc. | 48,23 | 4,56 | 7,04 | 32,14 |
| Tr. | 47,8 | 4,4 | 6,8 | 32,2 |

Exemple 24 : **5-O-[(3-t-butoxycarbonyl-4-L-thiazolidinyl) carbonyl]-, 2-O-nitro, 1,4:3,6-dianhydro-D-glucitol (24) :**

6 g (31,4 mmoles) de 1,4:3,6-dianhydro-D-glucitol-2-nitrate, 7,38 g (31,6 mmoles) de N-t-Boc-L-thioproline, 6,48 g (31,4 mmoles) de DCC, 4,26 g (31,5 mmoles) d'HOBt et 0,52 g (3,5 mmoles) de 4-pyrrolidinopyridine sont agités dans 270 ml de dichlorométhane (stabilisé sur amylène et séché sur alumine) pendant 24 heures. Après filtration du DCU, on ajoute 200 ml de dichlorométhane et la solution est lavée et séchée comme pour (1). Le produit isolé est recristallisé du mélange acétate d'éthyle-éther de pétrole, On obtient 7,6 g (Rdt = 60 %).
F = 93°C, $[\alpha]_D^{20}$= -15,6 (c 0,6 éthanol).

| Analyse | C | H | N |
|---|---|---|---|
| Calc. (0,15$H_2O$) | 44,03 | 5,48 | 6,84 |
| Tr. | 44,0 | 5,4 | 6,8 |

Exemple 25 : **5-O-[(4-L-thiazolidinyl)carbonyl]-, 2-O-nitro, 1,4:3,6-dianhydro-D-glucitol, monochlorhydrate (25) :**

A 65 ml d'une solution d'acide chlorhydrique 2 N dans l'acétate d'éthyle, on ajoute 6,8 g du composé précédent (24). La solution est agitée 1 heure à température ambiante et le précipité formé est filtré et lavé à l'éther avant d'être recristallisé dans le méthanol. On obtient 2,35 g (Rdt = 40 %).
F = 175°C (déc), $[\alpha]_D^{20}$= +15,8 (c 0,6 eau).

| Analyse | C | H | N |
|---|---|---|---|
| Calc. (0,15$H_2O$) | 34,75 | 4,45 | 8,10 |
| Tr. | 34,7 | 4,4 | 7,9 |

Exemple 26 : **L-cystéine, S-[(acétylamino)méthyl]-N-[(t-butoxy)carbonyl]-, ester du 2-O-nitro, 1,4:3,6-dianhydro-D-glucitol (26) :**

3,5 g (18 mmoles) de 1,4:3,6-dianhydro-D-glucitol-2-nitrate, 5,3 g (18 mmoles) de N-t-Boc-S acétamidométhyl-L-cystéine, 3,78 g (18 mmoles) de DCC, 2,43 g (18 mmoles) d'HOBt et 0,3 g (2 mmoles) de 4-pyrrolidinopyridine sont agités dans 150 ml de dichlorométhane (stabilisé sur amylène et séché sur alumine) pendant 24 heures. Après filtration du DCU, on ajoute 250 ml de dichlorométhane et la solution est lavée et séchée comme pour (1). Le produit isolé est recristallisé du mélange acétone-éther diisopropylique (40-60). On obtient 3,95 g (Rdt = 46 %).
F = 115°C, $[\alpha]_D^{20}$= +11,2 (c 0,6 éthanol).

| Analyse | C | H | N |
|---|---|---|---|
| Calc. | 43,86 | 5,84 | 9,02 |
| Tr. | 43,8 | 5,9 | 9,0 |

Example 27 : **L-cystéine, S-[(acétylamino)méthyl]-, ester du 2-O-nitro, 1,4:3,6-dianhydro-D-glucitol, monochlorhydrate (27) :**

A 21 ml d'une solution d'acide chlorhydrique 2 N dans l'acétate d'éthyl, on ajoute 2,3 g du composé précédent (26). La solution est agitée 1 heure à température ambiante et le précipité formé est filtré et lavé à l'éther avant d'être recristallisé dans l'éthanol. On obtient 1,75 g (Rgt = 88 %).
F = 161-162°C, $[\alpha]_D^{20}$= +31,6 (c 0,6 eau).

| Analyse | C | H | N |
|---|---|---|---|
| Calc. | 35,86 | 5,01 | 10,45 |
| Tr. | 35,4 | 4,9 | 10,3 |

Exemple 28 : **5-O-[2-[(phénylméthyl)thio]benzoyl]-, 2-O-nitro, 1,4:3,6-dianhydro-D-glucitol (28) :**

7,8 g (30 mmoles) de chlorure d'acide thiosalicylique S-benzylé sont dissous dans 65 ml de pyridine anhydre et introduits dans le réacteur. On coule alors goutte à goutte à 40°C une solution de 4 g (21 mmoles) de 1,4:3,6-dianhydro-D-glucitol-2-nitrate dans 35 ml de pyridine anhydre. Sous agitation, on laisse réagir pendant 24 heures. On traite alors comme pour (22). Le produit est recristallisé dans le mélange méthanol-acétone (40-20). On obtient 4,4 g de produit (Rdt = 51 %).
F = 105-106°C, $[\alpha]_D^{20}$= +77,2 (c 2, acétone).

| Analyse | C | H | N |
|---|---|---|---|
| Calc. | 57,55 | 4,59 | 3,35 |
| Tr. | 57,5 | 4,5 | 3,3 |

Exemple 29 : **Bis[5-O[(2-thio)benzoyl]-, 2-O-nitro, 1,4:3,6-dianhydro-D-glucitol] (29) :**

13,5 g (60 mmoles) d'acide acétamidométhyl thiosalicylique sont agités avec 50 ml de chlorure de thionyle pendant 1 heure à 70°C, on évapore sous vide le chlorure de thionyle en excès en évitant de dépasser 50°C. Après refroidissement, on ajoute 60 ml de pyridine sèche, ce qui provoque une vive effervescence et une montée en température. Par décantation, on récupère la solution noire et on y ajoute goutte à goutte une solution de 3,8 g (20 mmoles) de 1,4:3,6-dianhydro-D-glucitol-2-nitrate dans 10 ml de pyridine. On agite 30 heures à 40°C et on évapore la pyridine sous vide. On reprend au dichlorométhane, on filtre sur silice et la solution est lavée et séchée comme pour (1). Le produit récupéré est chromatographié sur colonne de silice. On isole 3,5 g de produit qui est recristallisé dans le mélange acétone-éthanol (1-2). On obtient 1,8 g (Rdt = 34 %) de produit.
F = 140-142°C, $[\alpha]_D^{20}$= +79,2 (c 0,5 acétone).

| Analyse | C | H | O | N | S |
|---|---|---|---|---|---|
| Calc. | 47,85 | 3,71 | 34,32 | 4,29 | 9,83 |
| Tr. | 47,8 | 3,3 | 34,3 | 4,0 | 9,6 |

Exemple 30 : **5-[[(4-L-thiazolidinyl)carbonyl]amino]-, 5-déoxy, 2-O-nitro, 1,4:3,6-dianhydro-L-iditol, monochlorhydrate (30) :**

∗ **Méthode A :**

On agite à 0°C une solution de 3,2 g (16,85 mmoles) de 1,4:3,6-dianhydro-5-déoxy-L-iditol-5-amino-2-nitrate dans 120 ml de dichlorométhane anhydre. On ajoute successivement 1,87 g (14,05 mmoles) de -L-thioproline, 2,9 g (14,05 mmoles) de DCC et 1,9 g (14,05 mmoles) d'HOBt. Après 3 heures à cette température, on agite encore 20 heures à température ambiante. Après filtration du DCU et évaporation du solvant, on chromatographie l'huile sur silice (230-400 mesch ASTM). L'huile isolée est solubilisée dans du dichlorométhane et on fait barboter du chlorure d'hydrogène. Après évaporation du solvant, le résidu est cristallisé avec le méthanol puis recristallisé dans ce solvant. On obtient 2,6 g (Rdt = 54 %).

∗ **Méthode B :**

On agite à température ambiante une solution de 5 g (26,3 mmoles) de 1,4:3,6-dianhydro-5-déoxy-L-iditol-5-amino-2-nitrate dans 300 ml de dichlorométhane anhydre. On ajoute successivement 6,13 g (26,3 mmoles) de N-t-Boc-L-thioproline, 5,43 g (26,3 mmoles) de DCC et 3,55 g (26,3 mmoles) d'HOBt. Après 24 heures de réaction et filtration du DCU, on ajoute 200 ml de dichlorométhane et on effectue les lavages comme pour (1). Le résidu d'évaporation est chromatographié sur silice (230-400 mesh ASTM) et on isole 10,5 g d'une huile correspondant au 5-[[3-t-butoxycarbonyl-4-L-thiazolidinyl)carbonyl] amino]-, 5-déoxy, 2-O-nitro, 1,4:3,6-dianhydro-L-iditol (50). Celle-ci n'a pas été cristallisée ; elle est traitée directement.

A 80 ml d'une solution d'acide chlorhydrique 2N dans l'acétate d'éthyle, on ajoute les 10,5 g du composé précédent. La solution est agitée 3 heures à température ambiante et le solide blanc formé est filtré puis lavé à l'éther. Après recristallisation dans le méthanol, on obtient 7,35 g de produit (30) (Rdt = 81 %).
F = 185-199°C (déc), $[\alpha]_D^{20}$= -60,7 (c 2, eau).

| Analyse | C | H | N | Cl |
|---|---|---|---|---|
| Calc. | 35,14 | 4,72 | 12,30 | 10,37 |
| Tr. | 35,1 | 4,5 | 12,1 | 10,4 |

Exemple 31 : **5-[[(3-formyl 2,2-diméthyl-4-L-thiazolidinyl)carbonyl]amino]-, 5-déoxy, 2-O-nitro, 1,4:3,6-dianhydro-L-iditol, monochlorhydrate (31) :**

On agite à température ambiante une solution de 2,94 g (15,5 mmoles) de 1,4:3,6-dianhydro-5-déoxy-L-iditol-5-amino-2-nitrate dans 150 ml de dichlorométhane anhydre. On ajoute successivement 2,95 g (15,5 mmoles) de N-formyl-L-diméthylthioproline, 3,20 g (15,5 mmoles) de DCC, 2,1 g (15,5 mmoles) d'HOBt. Après 24 heures de réaction, on filtre le DCU, évapore le solvant et chromatographie le solide obtenu (sur silice 230-400 mesh ASTM). Après recristallisation dans le méthanol, on obtient 3,05 g (Rdt = 54 %) de solide blanc.
F = 158°C, $[\alpha]_D^{20}$= -59 (c 0,6 acétone).

| Analyse | C | H | N |
|---|---|---|---|
| Calc. | 43,20 | 5,30 | 11,63 |
| Tr. | 43,0 | 5,3 | 11,7 |

Exemple 32 : **5-[[(2,2-diméthyl-4-L-thiazolidinyl)carbonyl]amino]-, 5-déoxy, 2-O-nitro, 1,4:3,6-dianhydro-L-iditol, monochlorhydrate (32) :**

On agite à température ambiante une solution de 3 g (15,8 mmoles) de 1,4:3,6-dianhydro-5-déoxy-L-iditol-5-amino-2-nitrate dans 130 ml de dichlorométhane anhydre. On ajoute successivement 3,07 g (15,5 mmoles) de -L-diméthylthioproline, 3,26 g (15,7 mmoles) de DCC et 2,14 g (15,7 mmoles) d'HOBt. Après 24 heures de réaction, on filtre le DCU, évapore le solvant et chromatographie le résidu huileux sur silice (230-400 mesch ASTM). On procède ensuite comme pour (30) et on obtient 2,7 g (Rdt = 47 %) de produit recristallisé dans le

méthanol.
F = 196°C (déc), $[\alpha]_D^{20}$= de -12 à +17 (c 0,6 eau) ;
variation due à l'instabilité du produit dans l'eau ; il se transforme en :

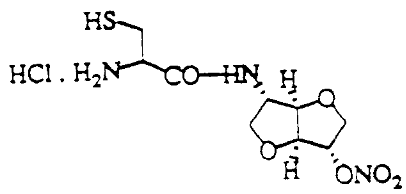

qui évolue lui-même vers la forme oxydée (cystine).

| Analyse | C | H | N | Cl |
|---|---|---|---|---|
| Calc. | 38,97 | 5,45 | 11,36 | 9,58 |
| Tr. | 38,5 | 5,4 | 11,1 | 9,5 |

Exemple 33 : **L-cystéine, S-[(acétylamino)méthyl]-N-[(t-butoxy)carbonyl]-, amide du 5-amino, 5-déoxy, 2-O-nitro, 1,4:3,6-dianhydro-L-iditol (33) :**

On agite à température ambiante une solution de 2,56 g (13,5 mmoles) de 1,4:3,6-dianhydro-5-déoxy-L-iditol-5-amino-2-nitrate dans 120 ml de dichlorométhane anhydre. On ajoute successivement 3,95 g (13,5 mmoles) de N-t-Boc-S-acétamidométhyl-L-cystéine, 2,8 g (13,5 mmoles) de DCC, 1,82 g (13,5 mmoles) d'HOBt. Après 24 heures de réaction, filtration du DCU et évaporation du solvant, on chromatographie sur silice le composé pâteux isolé (230400 mesh ASTM). On recristallise le solide obtenu dans l'acétate d'éthyle. On obtient 5,9 g (Rdt = 94 %).
F = 152°C, $[\alpha]_D^{20}$= +26,1 (c 0,6 acétone).

| Analyse | C | H | N |
|---|---|---|---|
| Calc. | 43,96 | 6,07 | 12,06 |
| Tr. | 44,1 | 6,0 | 12,0 |

Exemple 34 : **L-cystéine, S-[(acétylamino)méthyl]-, amide du 5-amino, 5-déoxy, 2-O-nitro, 1,4:3,6-dianhydro-L-iditol, monochlorhydrate (34) :**

A 13 ml d'une solution d'acide chlorhydrique 1,6 N dans le méthanol, on ajoute 2 g du composé précédent (33). La solution est agitée 24 heures à température ambiante et on filtre le solide blanc. On évapore le solvant du filtrat et on chromatographie le composé visqueux obtenu (silice 230-400 mesh ASTM). On cristallise l'huile isolée avec de la pyridine et de l'acétone et le solide est recristallisé du mélange éthanol-acétone, puis dans l'éthanol. On obtient 0,95 g (Rdt = 55 %).
F = 183°C, $[\alpha]_D^{20}$= +28,3 (c 0,6 eau).

| Analyse | C | H | N |
|---|---|---|---|
| Calc. (0,15 $H_2O$) | 35,71 | 5,31 | 13,88 |
| Tr. | 35,7 | 5,3 | 13,7 |

Exemple 35 : **L-cystéine, S-[(2-nitro-pnényl)thio]-N-[(t-butoxy)carbonyl]-, amide du 5-amino, 5-déoxy, 2-O-nitro, 1,4:3,6-dianhydro-L-iditol (35) :**

A une solution de 2 g (4,3 mmoles) de (33) dans 12 ml d'acide acétique anhydre, on coule, à température ambiante et sous agitation, une solution de 1 g (5, 2 mmoles) de chlorure de 2-nitrobenzènesulfényle. Après 2 heures 30 de réaction, on évapore l'acide acétique sous vide. L'huile obtenue est cristallisée à l'éther, puis on chromatographie le produit sur silice (230-400 mesh ASTM). Le solide isolé est recristallisé dans le méthanol. On obtient 1,1 g (Rdt = 38 %).
F = 157-158°C, $[\alpha]_D^{20}$= +5,33 (c 0,6 acétone).

| Analyse | C | H | N |
|---|---|---|---|
| Calc. | 43,95 | 4,79 | 10,25 |
| Tr. | 43,9 | 4,7 | 10,2 |

Dans les conditions de la réaction, un sous-produit est obtenu en quantité importante ; il correspond au sel de l'amine (36) formé après départ du groupement t-butoxy-carbonyle.

Exemple 36 : **L-méthionine, N-[(t-butoxy)carbonyl]-, amide du 5-amino, 5-déoxy, 2-O-nitro, 1,4:3,6-dianhydro-L-iditol (37) :**

On agite à température ambiante une solution de 3,85 g (22 mmoles) de 1,4:3,6-dianhydro-5-déoxy-L-iditol-5-amino-2-nitrate dans 190 ml de dichlorométhane anhydre. On ajoute successivement 5 g (20 mmoles) de N-t-Boc-L-méthionine, 4,15 g (21 mmoles) de DCC et 2,7 g (21 mmoles) d'HOBt. Après 24 heures de réaction, on filtre le DCU, on évapore le solvant et on chromatographie l'huile obtenue (silice 230-400 mesh ASTM). Le composé isolé est recristallisé dans le mélange acétate d'éthyle-éther diisopropylique (1-2). On obtient 4,7 g (Rdt = 55 %).
F = 96°C.

Le traitement de ce composé par une solution d'acide chlorhydrique 1,7 N dans le méthanol permet d'enlever le groupement t-butoxy-carbonyle et conduit au chlorhydrate d'amine correspondant (38).

Exemple 37 : **5-[[2-[(phénylméthyl)thio]benzoyl]amino]-, 5-déoxy, 2-O-nitro, 1,4:3,6-dianhydro-L-iditol (39) :**

On agite à température ambiante une solution de 3 g (15,8 mmoles) de 1,4:3,6-dianhydro-5-déoxy-L-iditol-5-amino-2-nitrate dans 130 ml de dichlorométhane anhydre. On ajoute successivement 3,2 g (13,1 mmoles) d'acide thiosalicylique S-benzylé et 2,75 g (13,3 mmoles) de DCC. Après 24 heures de réaction, on filtre le DCU, on évapore le solvant et on chromatographie l'huile obtenue (silice 230-400 mesh ASTM). Après recristallisation du mélange acétone-hexane, on obtient 2,55 g (Rdt = 47 %).
F = 87-88°C, $[\alpha]_D^{20}$= +29,3 (c 2 acétone).

| Analyse | C | H | N |
|---|---|---|---|
| Calc. | 57,68 | 4,84 | 6,72 |
| Tr. | 57,6 | 5,0 | 6,7 |

Exemple 38 : **2-[[(4-L-thiazolidinyl)carbonyl]amino]-, 2-déoxy, 5-O-nitro, 1,4:3,6-dianhydro-D-glucitol, monochlorhydrate (40) :**

On agite à température ambiante une solution de 4,45 g (23,4 mmoles) de 1,4:3,6-dianhydro-2-déoxy-D-glucitol-2-amino-5-nitrate dans 200 ml de dichlorométhane anhydre. On ajoute successivement 3,1 g (23,3 mmoles) de -L-thioproline, 4,8 g (23,3 mmoles) de DCC et 3,15 g (23,3 mmoles) d'HOBt. Après 24 heures de réaction, on filtre le DCU, on évapore le solvant et on chromatographie le composé obtenu (silice 230-400 mesh ASTM). On procède ensuite comme pour (30) et on recristallise du méthanol le chlorhydrate obtenu. On isole 2,3 g (Rdt = 29 %).
F = 187-188°C (déc), $[\alpha]_D^{20}$= +11,8 (c 0,6 eau).

| Analyse | C | H | N | S | Cl |
|---|---|---|---|---|---|
| Calc. | 35,14 | 4,72 | 12,30 | 9,38 | 10,37 |
| Tr. | 35,2 | 4,7 | 11,7 | 9,2 | 10,3 |

Exemple 39 : **L-cystéine, S-[(acétylamino)méthyl]-N-[(t-butoxy)carbonyl]-, amide du 2-amino, 2-déoxy, 5-O-nitro, 1,4:3,6 -dianhydro-D-glucitol (41) :**

On agite à température ambiante une solution de 4,15 g (21,8 mmoles) de 1,4:3,6-dianhydro-2-déoxy-D-glucitol-2-amino-5-nitrate dans 200 ml de dichlorométhane anhydre. On ajoute successivement 6,4 g (21,8 mmoles) de N-t-Boc-S-acétamidométhyl-L-cystéine, 4,53 g (21,9 mmoles) de DCC et 2,95 g (21,8 mmoles) d'HOBt. Après 24 heures de réaction, filtration du DCU et évaporation du solvant, on chromatographie sur silice (230-400 mesh ASTM). Le composé isolé est recristallisé dans l'acétate d'éthyle ; on obtient 4,5 g (Rdt = 44 %).

F = 90-92°C, $[\alpha]_D^{20}$= +78,1 (c 0,6 éthanol).

| Analyse | C | H | N | O |
|---|---|---|---|---|
| Calc. (0,6 $H_2O$) | 42,96 | 6,18 | 11,78 | 32,31 |
| Tr. | 42,9 | 6,2 | 11,7 | 32,4 |

Exemple 40 : **L-cystéine, S-[(acétylamino)méthyl]-, amide du 2-amino, 2-déoxy, 5-O-nitro, 1,4:3,6-dianhydro-D-glucitol, monochlorhydrate (42) :**

A 23 ml d'une solution d'acide chlorhydrique 2,2 N dans le méthanol, on ajoute 4,3 g de composé précédent (41). On agite 18 heures à température ambiante et on concentre la solution avant de la chromatographier sur silice (230-400 mesh ASTM). Le composé isolé est recristallisé dans l'éthanol ; on obtient 3,1 g (Rdt = 83 %).

F = 94-95°C, $[\alpha]_D^{20}$= +74,8 (c 0,6 eau).

| Analyse | C | H | N | Cl |
|---|---|---|---|---|
| Calc. (0,8 $H_2O$) | 34,71 | 5,23 | 13,49 | 8,53 |
| Tr. | 34,7 | 5,4 | 13,3 | 8,6 |

Exemple 41 : **L-méthionine, N-[(t-butoxy)carbonyl]-, amide du 2-amino, 2-déoxy, 5-O-nitro, 1,4:3,6-dianhydro-D-glucitol (43) :**

On agite à température ambiante une solution de 3,26 g (18 mmoles) de 1,4:3,6-dianhydro-2-déoxy-D-glucitol-2-amino-5-nitrate dans 170 ml de dichlorométhane anhydre. On ajoute successivement 4,5 g (18 mmoles) de N-t-Boc-L-méthionine, 7,65 g (18 mmoles) de CMC et 2,42 g (18 mmoles) d'HOBt. Après 24 heures de réaction, on ajoute du dichlorométhane et on procède comme pour la préparation de (1). On isole 5,4 g d'un solide qui est recristallisé dans le mélange éthanol-éther diisopropylique (1-4). On obtient 2,55 g (Rdt = 34 %).

F = 114°C, $[\alpha]_D^{20}$= +67,6 (c 0,6 éthanol).

| Analyse | C | H | N |
|---|---|---|---|
| Calc. | 45,59 | 6,45 | 9,96 |
| Tr. | 45,3 | 6,3 | 9,8 |

Le traitement de ce composé par une solution d'acide chlorhydrique 1,7 N dans le méthanol conduit au chlorhydrate correspondant (44).

Exemple 42 : **2-[[2-[(phénylméthyl)thio]benzoyl]amino]-, 2-déoxy, 5-O-nitro, 1,4:3,6-dianhydro-D-glucitol (45) :**

On agite à température ambiante une solution de 3,5 g (18,4 mmoles) de 1,4:3,6-dianhydro-2-déoxy-D-glucitol-2-amino-5-nitrate dans 160 ml de dichlorométhane anhydre. On ajoute successivement 4,5 g (18,4 mmoles) d'acide thiosalicylique S-benzylé et 3,8 g (18,4 mmoles) de DCC. Après 24 heures de réaction, filtration du DCU et évaporation du solvant, on chromatographie le résidu sur silice (230-400 mesh ASTM) et on recristallise le composé isolé dans le mélange acétate d'éthyle-éther diisopropylique. On obtient 2,2 g (Rdt = 36 %).
F = 86-90°C, $[\alpha]_D^{20}$= +64,8 (c 0,6 éthanol).

| Analyse | C | H | N |
|---|---|---|---|
| Calc. | 57,68 | 4,84 | 6,72 |
| Tr. | 57,7 | 4,8 | 6,6 |

Exemple 43 : **5-[[(3-acétyl-4-L-thiazolidinyl) carbonyl] amino]-, 5-déoxy, 2-O-nitro, 1,4:3,6-dianhydro-L-iditol (51) :**

On agite à température ambiante une solution de 2 g (10,5 mmoles) de 1,4:3,6-dianhydro-5-déoxy-L-iditol-5-amino-2-nitrate dans 80 ml de dichlorométhane anhydre. On ajoute successivement 1,84 g (10,5 mmoles) de N-acétyl-L-thioproline, 4,45 g (10,5 mmoles) de CMC et 1,42 g (10,5 mmoles) d'HOBt. Après 24 heures de réaction, on ajoute 50 ml de dichlorométhane et on effectue les lavages comme pour (1). Après séchage de la phase organique sur sulfate de sodium, filtration et évaporation du solvant, le solide obtenu est recristallisé dans l'acétate d'éthyle. On obtient 1, 6 g de produit ( Rdt = 44 % ).
F = 169°C, $[\alpha]_D^{20}$= -62,0 (c 1, éthanol).

| Analyse | C | H | N |
|---|---|---|---|
| Calc. | 41,50 | 4,93 | 12,10 |
| Tr. | 41,4 | 4,9 | 12,1 |

Exemple 44 : **5-[[(3-benzoyl-4-L-thiazolidinyl) carbonyl] amino]-, 5-déoxy, 2-O-nitro, 1,4:3,6-dianhydro-L-iditol (52) :**

On agite à température ambiante une solution de 2 g (10,5 mmoles) de 1,4:3,6-dianhydro-5-déoxy-L-iditol-5-amino-2-nitrate dans 100 ml de dichlorométhane anhydre. On ajoute successivement 2,5 g (10,5 mmoles) de N-benzoyl-L-thioproline, 2,16 g (10,5 mmoles) de DCC et 1,42 g (10,5 mmoles) d'HOBt. Après 24 heures de réaction, on filtre le DCU, on ajoute 100 ml de dichlorométhane et on effectue les lavages comme pour (1). Le résidu obtenu après évaporation est chromatographié sur silice (230-400 mesh ASTM) et le produit isolé est recristallisé dans le méthanol ; on obtient 2,04 g de composé (Rdt = 40 %).
F = 166-167°C, $[\alpha]_D^{20}$= -106,9 (c 1, DMSO).

| Analyse | C | H | N |
|---|---|---|---|
| Calc. | 49,87 | 4,68 | 10,26 |
| Tr. | 49,7 | 4,6 | 10,2 |

Exemple 45 : **5-[[(3-t-butoxycarbonyl-2-méthyl-4-L-thiazolidinyl) carbonyl] amino]-, 5-déoxy, 2-O-nitro, 1,4:3,6-dianhydro-L-iditol (53) :**

On agite à température ambiante une solution de 3 g (15,7 mmoles) de 1,4:3,6-dianhydro-5-déoxy-L-iditol-5-amino-2-nitrate dans 120 ml de dichlorométhane anhydre. On ajoute successivement 3, 9 g (15,7 mmoles)

de N-t-Boc-2-méthyl-L-thioproline, 3,26 g (15,7 mmoles) de DCC et 2, 13 g (15,7 mmoles) d'HOBt. Après 24 heures de réaction et filtration du DCU, on ajoute 80 ml de dichlorométhane et on effectue les lavages comme pour (1). Le résidu obtenu après évaporation est chromatographié sur silice (230-400 mesh ASTM). On isole 5,6 g d'un solide blanc qui est recristallisé dans l'acétate d'éthyle. On obtient 4,17 g de produit (Rdt = 63 %). La RMN indique une seule configuration pour le carbone 2.
F = 129-130°C, $[\alpha]_D^{20}$= -18,8 (c 1, éthanol).

| Analyse | C | H | N |
|---|---|---|---|
| Calc. | 45,82 | 6,01 | 10,02 |
| Tr. | 45,7 | 6,0 | 10,0 |

Exemple 46 : **5-[[(2-méthyl-4-L-thiazolidinyl) carbonyl] amino]-, 5-déoxy, 2-O-nitro, 1,4:3,6-dianhydro-L-iditol, monochlorhydrate (54) :**

A 34 ml d'une solution d'acide chlorhydrique 2N dans l'acétate d'éthyle, on ajoute 4,09 g du composé précédent. La solution est agitée 16 heures à température ambiante et le solide blanc formé est filtré puis lavé à l'éther. Après recristallisation dans le méthanol, on obtient 2,58 g de produit (Rdt = 74 %). La RMN montre qu'il correspond à un mélange de deux diastéréoisomères en 2.
F = 209°C (déc.), $[\alpha]_D^{20}$= -55,1 (c 1, eau).

| Analyse | C | H | N | Cl |
|---|---|---|---|---|
| Calc. | 37,13 | 5,10 | 11,81 | 9,96 |
| Tr. | 37,2 | 5,1 | 11,8 | 9,9 |

Exemple 47 : **5-[[(3-t-butoxycarbonyl-2-méthoxycarbonyl-4-L-thiazolidinyl) carbonyl] amino]-, 5-déoxy, 2-O-nitro, 1,4:3,6-dianhydro-L-iditol (55) :**

On agite à température ambiante une solution de 1,73 g (9,1 mmoles) de 1,4:3,6-dianhydro-5-déoxy-L-iditol-5-amino-2-nitrate dans 50 ml de dichlorométhane anhydre. On ajoute successivement 2,66 g (9,1 mmoles) de N-t-Boc-2-méthoxycarbonyl-L-thioproline, 1,89 g (9,1 mmoles) de DCC et 1,23 g (9,1 mmoles) d'HOBt. Après 24 heures de réaction et filtration du DCU, on ajoute 50 ml de dichlorométhane et on effectue les lavages comme pour (1). Le résidu huileux obtenu après évaporation est chromatographié sur silice (230-400 mesh ASTM). On isole 2,44 g d'un solide blanc qui est recristallisé dans le méthanol. On obtient 2,04 g de produit (Rdt = 48 %). La RMN indique une seule configuration pour le carbone 2.
F = 132-133°C, $[\alpha]_D^{20}$= -13,9 (c 1, éthanol).

| Analyse | C | H | N |
|---|---|---|---|
| Calc. | 44,06 | 5,44 | 9,07 |
| Tr. | 44,2 | 5,6 | 9,2 |

Exemple 48 : **5-[[3-t-butoxycarbonyl-2-phényl-4-L-thiazolidinyl) carbonyl] amino]-, 5-déoxy, 2-O-nitro, 1,4:3,6-dianhydro-L-iditol (56) :**

On agite à température ambiante une solution de 5 g (26,29 mmoles) de 1,4:3,6-dianhydro-5-déoxy-L-iditol-5-amino-2-nitrate dans 200 ml de dichlorométhane anhydre. On ajoute successivement 8,1 g (26,29 mmoles) de N-t-Boc-2-phényl-L-thioproline, 5,4 g (26,29 mmoles) de DCC et 3,6 g (26,29 mmoles) d'HOBt. Après 24 heures de réaction et filtration du DCU, on ajoute 100 ml de dichlorométhane et on effectue les lavages comme pour (1). Le résidu huileux obtenu après évaporation est chromatographié sur silice (230-400 mesh ASTM). On isole 10,7 g d'un solide blanc qui est recristallisé dans l'acétate d'éthyle. On obtient 9,3 g de produit (Rdt = 73 %). La RMN indique une seule configuration pour le carbone 2.

F = 115°C, $[\alpha]_D^{20}$= +87,5 (c 1, éthanol).

| Analyse | C | H | N |
|---|---|---|---|
| Calc. (0,5 $H_2O$) | 51,42 | 5,74 | 8,56 |
| Tr. | 51,3 | 5,6 | 8,6 |

Exemple 49 : **5-[[(2-phényl-4-L-thiazolidinyl) carbonyl] amino]-, 5-déoxy, 2-O-nitro, 1,4:3,6-dianhydro-L-iditol, monochlorhydrate (57) :**

A 15 ml d'une solution d'acide chlorhydrique 2N dans l'acétate d'éthyle, on ajoute 2 g du composé précédent. La solution est agitée 16 heures à température ambiante et après évaporation du solvant, on récupère 1,66 g d'un solide blanc. Après recristallisation dans le méthanol, on obtient 0,95 g de produit (Rdt = 50 %). La RMN montre qu'il correspond à un mélange de deux diastéréoisomères en 2.
F = 197°C (déc.), $[\alpha]_D^{20}$= -38,1 (c 1, DMSO).

| Analyse | C | H | N | Cl |
|---|---|---|---|---|
| Calc. (0,6 $H_2O$) | 44,83 | 4,98 | 9,80 | 8,27 |
| Tr. | 44,5 | 4,7 | 9,6 | 8,4 |

Exemple 50 : **5-[[(3-t-butoxycarbonyl-2-butyl-4-L-thiazolidinyl) carbonyl] amino]-, 5-déoxy, 2-O-nitro, 1,4:3,6-dianhydro-L-iditol (58) :**

On agite à température ambiante une solution de 4 g (21 mmoles) de 1,4:3,6-dianhydro-5-déoxy-L-iditol-5-amino-2-nitrate dans 100 ml de dichlorométhane anhydre. On ajoute successivement 6,08 g (21 mmoles) de N-t-Boc-2-butyl-L-thioproline, 4,34 g (21 mmoles) de DCC et 2,84 g (21 mmoles) d'HOBt. Après 24 heures de réaction et filtration du DCU, on ajoute 120 ml de dichlorométhane et on effectue les lavages comme pour (1). Le résidu huileux obtenu après évaporation est chromatographié sur silice (230-400 mesh ASTM). On isole 7,8 g d'un solide blanc qui est recristallisé dans un mélange d'acétate d'éthyle et d'éther de pétrole. On obtient 4,76 g de produit (Rdt = 49 %). La RMN indique une seule configuration pour le carbone 2.
F = 91-92°C, $[\alpha]_D^{20}$= +4,3 (c 1, éthanol).

| Analyse | C | H | N |
|---|---|---|---|
| Calc. | 49,44 | 6,77 | 9,11 |
| Tr. | 49,7 | 6,7 | 9,1 |

Exemple 51 : **5-[[(2-butyl-4-L-thiazolidinyl) carbonyl] amino]-, 5-déoxy, 2-O-nitro, 1,4:3,6-dianhydro-L-iditol, monochlorhydrate (59) :**

A 28 ml d'une solution d'acide chlorhydrique 2N dans l'acétate d'éthyle, on ajoute 3,8 du composé précédent. La solution est agité 16 heures à température ambiante et le solide blanc formé est filtré, puis lavé à l'éther. Après recristallisation dans le méthanol, on obtient 2,33 g de produit (Rdt = 71 %). La RMN montre qu'il correspond à un mélange de deux diastéréoisomères en 2.
F = 198-200°C (déc.), $[\alpha]_D^{20}$= -34,2 (c 1, DMSO).

| Analyse | C | H | N | Cl |
|---|---|---|---|---|
| Calc. | 42,31 | 6,09 | 10,58 | 8,81 |
| Tr. | 42,1 | 6,0 | 10,4 | 8,6 |

## III - SYNTHESE DES COMPOSES DE FORMULE I avec n = 1.

**III-a : R-CO + A-Y-B :** Le Tableau III ci-après montre les différents dérivés synthétisés.

TABLEAU III

| A-Y-B \ R | 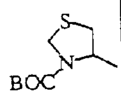 | 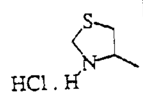 | 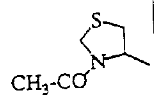 | 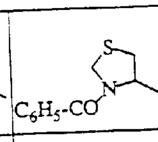 | 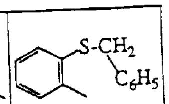 |
|---|---|---|---|---|---|
| 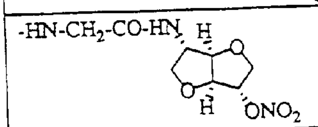 | 60 | 46 | 75 | 76 | 47 |
| 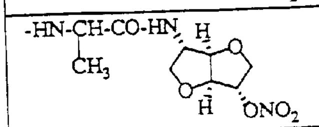 | 61 | 62 | | | |
| 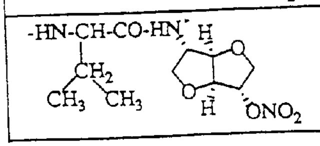 | 63 | 64 | | | |

TABLEAU III

| R / A-Y-B | 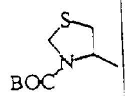 | 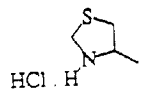 | 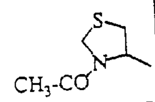 | 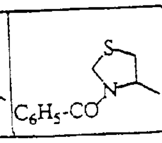 | 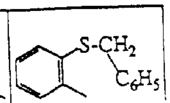 |
|---|---|---|---|---|---|
| 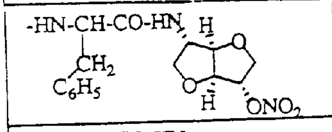 | 65 | 66 | | | |
| 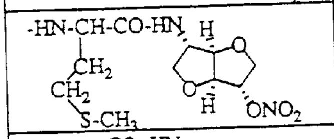 | 67 | 68 | | | |
| 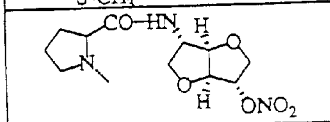 | 69 | 70 | | | |
| 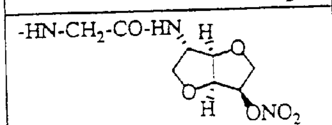 | | 71 | | | |
| 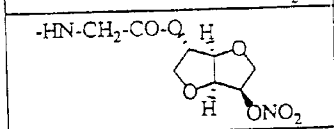 | 72 | 73 | | | |
| 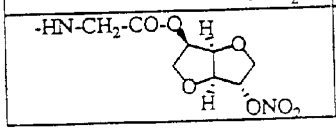 | | 74 | | | |

Exemple 52 : **5-[[2-[[(3-t-butoxycarbonyl-4-L-thiazolidinyl) carbonyl] amino] 1-oxo éthyl] amino]-, 5-déoxy, 2-O-nitro, 1,4:3,6-dianhydro-L-iditol (60) :**

On agite à température ambiante une solution de 1 g (4,05 mmole) de la forme base correspondant à (6) dans 35 ml de dichlorométhane anhydre. On ajoute successivement 0,94 g (4,05 mmoles) de N-t-Boc-L-thioproline, 0,83 g (4,05 mmoles) de DCC et 0,55 g (4,05 mmoles) d'HOBt. Après 24 heures de réaction, on filtre le DCU et évapore le solvant. On chromatographie l'huile sur silice (230-400 mesh ASTM) et on isole 1,8 g d'une huile qui cristallise. Le solide obtenu est recristallisé dans le mélange acétate d'éthyle-éther diisopropylique, puis lavé à l'éther de pétrole. On obtient 1,33 g de produit (Rdt = 71 %).
F = 127-129°C, $[\alpha]_D^{20}$= -38,3 (c 0,6, éthanol).

| Analyse | C | H | N |
|---|---|---|---|
| Calc. | 44,15 | 5,66 | 12,11 |
| Tr. | 43,8 | 5,6 | 12,0 |

Exemple 53 : **5-[[2-[[(4-L-thiazolidinyl)carbonyl] amino] 1-oxo éthyl] amino]-, 5-déoxy, 2-O-nitro, 1,4:3,6-dianhydro-L-iditol, monochorhydrate (46) :**

∗ **Méthode A :**

A 132 ml d'une solution d'acide chlorhydrique 2N dans l'acétate d'éthyle, on ajoute 18 g du composé précédent (60). La solution est agitée 16 heures à température ambiante et le solide blanc formé est filtré, puis lavé à l'éther ; en concentrant les eaux mères, on récupère un deuxième jet de produit qui est ajouté au précédent. Après recristallisation dans le méthanol, on obtient 11,9 g de produit (Rdt = 76 %).
F = 183-188°C (déc), $[\alpha]_D^{20}$= -46,9 (c 1, eau).

| Analyse | C | H | N |
|---|---|---|---|
| Calc. | 36,14 | 4,80 | 14,04 |
| Tr. | 36,1 | 4,8 | 14,1 |

∗ **Méthode B :**

On agite à température ambiante une solution de 2 g (8,09 mmoles) de la forme base correspondant à (6) dans 70 ml de dichlorométhane anhydre. On ajoute successivement 1,07 g (8 mmoles) de -L-thioproline, 1,65 g (8 mmoles) de DCC et 1,09 g (8 mmoles) d'HOBt. Après 24 heures de réaction, on filtre le DCU et on évapore le solvant. On chromatographie l'huile sur silice (230-400 mesh ASTM) et on isole un composé qui est transformé en son chlorhydrate, comme pour (30) ; le solide est chauffé à reflux dans l'acétate d'éthyle, filtré et recristallisé du méthanol. On obtient 2,15 g (Rdt = 67 %).
F = 183°C (déc), $[\alpha]_D^{20}$= -46,9 (c 1 eau).

| Analyse | C | H | N |
|---|---|---|---|
| Calc. | 36,14 | 4,80 | 14,04 |
| Tr. | 36,0 | 4,8 | 13,9 |

Exemple 54 : **5-[[2-[[(3-t-butoxycarbonyl-4-L-thiazolidinyl) carbonyl] amino] 1-(2-méthyl (2S))-oxo éthyl] amino]-, 5-déoxy, 2-O-nitro, 1,4:3,6-dianhydro-L-iditol (61) :**

On agite à température ambiante une solution de 5 g (19,1 mmoles) de la forme base correspondant à (8) dans,190 ml de dichlorométhane anhydre. On ajoute successivement 4,5 g (19,1 mmoles) de N-t-Boc-L-thioproline, 4 g (19,1 mmoles) de DCC et 2,6 g (19,1 mmoles) d'HOBt. Après 24 heures de réaction, on filtre le DCU et évapore le solvant. On chromatographie l'huile sur silice (230-400 mesh ASTM) et on isole une huile qui est solubilisée à chaud dans l'acétate d'éthyle ; on cristallise ainsi le DCU restant. On ajoute au filtrat l'éther diisopropylique, ce qui permet de récupérer 6,5 g de composé attendu. Le solide ainsi obtenu est recristallisé deux fois dans le mélange acétate d'éthyle-éther diisopropylique. On obtient 4,8 g de produit (Rdt = 52 %).
F = 150°C, $[\alpha]_D^{20}$= -77,5 (c 1, éthanol).

| Analyse | C | H | N |
|---|---|---|---|
| Calc. | 45,37 | 5,92 | 11,76 |
| Tr. | 45,2 | 5,9 | 11,8 |

Exemple 55 : **5-[[2-[[(4-L-thiazolidinyl) carbonyl] amino] 1-(2-méthyl (2S))-oxo éthyl] amino]-, 5-déoxy, 2-O-nitro, 1,4:3,6-dianhydro-L-iditol, monochlorhydrate (62) :**

A 14 ml d'une solution d'acide chlorhydrique 2N dans L'acétate d'éthyle, on ajoute 2 g du composé précédent (61). La solution est agitée 5 heures à température ambiante et le solide blanc formé est filtré puis lavé

à l'éther. Après recristallisation dans l'éthanol puis dans le méthanol, on obtient 0,95 g de produit (Rdt = 54 %).
F = 185-186°C (déc), $[\alpha]_D^{20}$= -75,0 (c 1, eau).

| Analyse | C | H | N | Cl |
|---|---|---|---|---|
| Calc. (0,3 H2O) | 37,30 | 5,19 | 13,39 | 8,47 |
| Tr. | 37,3 | 5,1 | 13,3 | 8,6 |

Exemple 56 : **5-[[2-[[(3-t-butoxycarbonyl-4-L-thiazolidinyl) carbonyl] amino] 1-(2-isopropyl (2S))-oxo éthyl] amino]-, 5-déoxy, 2-O-nitro, 1,4:3,6-dianhydro-L-iditol (63) :**

On agite à température ambiante une solution de 2 g (6,9 mmoles) de la forme base correspondant à (10) dans 70 ml de dichlorométhane anhydre. On ajoute successivement 1,63 g (6,9 mmoles) de N-t-Boc-L-thioproline, 1,43 g (6,9 mmoles) de DCC et 0,95 g (6,9 mmoles) d'HOBt. Après 20 heures de réaction et filtration du DCU, on ajoute 80 ml de dichlorométhane et on effectue les lavages comme pour (1). Le résidu huileux obtenu après évaporation est chromatographié sur silice (230-400 mesh ASTM) et on isole 2,55 g d'une pâte blanche qui est recristallisée dans le mélange acétate d'éthyle-éther diisopropylique. On obtient 1,95 g de produit (Rdt = 56 %).
F = 108-109°C, $[\alpha]_D^{20}$= -57,5 (c 0,6, éthanol).

| Analyse | C | H | N |
|---|---|---|---|
| Calc. | 47,61 | 6,39 | 11,10 |
| Tr. | 47,5 | 6,4 | 11,2 |

Exemple 57 : **5-[[2-[[(4-L-thiazolidinyl) carbonyl] amino] 1-(2-isopropyl (2S))-oxo éthyl] amino]-, 5-déoxy, 2-O-nitro, 1,4:3,6-dianhydro-L-iditol, monochlorhydrate (64) :**

A 50 ml d'une solution d'acide chlorhydrique 2N dans l'acétate d'éthyle, on ajoute 5,9 g du composé précédent (63). La solution est agitée 3 heures à température ambiante et le solide blanc formé est filtré puis lavé à l'éther. Après recristallisation dans l'éthanol, on obtient 3 g de produit (Rdt = 58 %).
F = 169°C (déc), $[\alpha]_D^{20}$= -60,7 (c 1, eau).

| Analyse | C | H | N | Cl |
|---|---|---|---|---|
| Calc. | 40,86 | 5,71 | 12,70 | 8,04 |
| Tr. | 40,9 | 5,6 | 12,6 | 8,0 |

Exemple 58 : **5-[[2-[[(3-t-butoxycarbonyl-4-L-thiazolidinyl) carbonyl] amino] 1-(2-benzyl (2S))-oxo éthyl] amino]-, 5-déoxy, 2-O-nitro, 1,4:3,6-dianhydro-L-iditol (65) :**

On agite à température ambiante une solution de 5 g (14,8 mmoles) de la forme base correspondant à (12) dans 200 ml de dichlorométhane anhydre. On ajoute successivement 3,45 g (14,8 mmoles) de N-t-Boc-L-thioproline, 3,05 g (14,8 mmoles) de DCC et 2 g (14,8 mmoles) d'HOBt. Après 20 heures de réaction et filtration du DCU, on ajoute 100 ml de dichlorométhane et on effectue les lavages comme pour (1). Le résidu pâteux obtenu après évaporation est chromatographié sur silice (230-400 mesh ASTM) et on isole 8,5 g d'une pâte blanche qui est recristallisée dans l'acétate d'éthyle. On obtient 5,2 g de produit (Rdt = 63 %).
F = 139-140°C, $[\alpha]_D^{20}$= -68,7 (c 1, éthanol).

| Analyse | C | H | N |
|---|---|---|---|
| Calc. | 52,17 | 5,84 | 10,14 |
| Tr. | 52,3 | 5,9 | 10,1 |

Exemple 59 : **5-[[2-[[(4-L-thiazolidinyl) carbonyl] amino] 1-(2-benzyl (2S))-oxo éthyl] amino]-, 5-déoxy, 2-O-nitro, 1,4:3,6-dianhydro-L-iditol, monochlorhydrate (66) :**

A 23 ml d'une solution d'acide chlorhydrique 2N dans l'acétate d'éthyle, on ajoute 3,65 g du composé précédent (65). La solution est agitée 15 heures à température ambiante et le solide pâteux formé est récupéré, lavé à l'acétate d'éthyle puis à l'éther. Après recristallisation dans l'éthanol, on obtient 1,8 g de produit (Rdt = 55 %).
F = 142-152°C (déc), $[\alpha]_D^{20}$= -25,0 (c 1, eau).

| Analyse | C | H | N | Cl |
|---|---|---|---|---|
| Calc. (0,8 $H_2O$) | 43,33 | 5,32 | 11,1 | 7,04 |
| Tr. | 45,1 | 5,0 | 11,5 | 6,9 |

Exemple 60 : **5-[[2-[[(3-t-butoxycarbonyl-4-L-thiazolidinyl) carbonyl] amino] 1-(2-(2′-thiométhyl) éthyl (2S))-oxo éthyl] amino]-, 5-déoxy, 2-O-nitro, 1,4:3,6-dianhydro-L-iditol (67) :**

On agite à température ambiante une solution de 7,2 g (22,4 mmoles) de la forme base correspondant à (38) dans 220 ml de dichlorométhane anhydre. On ajoute successivement 5,22 g (22,4 mmoles) de N-t-Boc-L-thioproline, 4,62 g (22,4 mmoles) de DCC et 3,03 g (22,4 mmoles) d'HOBt. Après 20 heures de réaction, on filtre le DCU et évapore le solvant. Le résidu huileux est chromatographié sur silice (230-400 mesh ASTM) et on isole 11,9 g d'un solide blanc qui est recristallisé dans le mélange acétate d'éthyle-éther diisopropylique. On obtient 9,90 g de produit (Rdt = 82 %).
F = 141°C, $[\alpha]_D^{20}$= -54,66 (c 0,6, éthanol).

| Analyse | C | H | N |
|---|---|---|---|
| Calc. | 44,76 | 6,01 | 10,44 |
| Tr. | 44,9 | 6,0 | 10,3 |

Exemple 61 : **5-[[2-[[(4-L-thiazolidinyl) carbonyl] amino] 1-(2-(2′-thiométhyl) éthyl (2S))-oxo éthyl] amino]-, 5-déoxy, 2-O-nitro, 1,4:3,6-dianhydro-L-iditol, monochlorhydrate (68) :**

A 68 ml d'une solution d'acide chlorhydrique 1N dans l'acétate d'éthyle, on ajoute 8,5 g du composé précédent (67). La solution est agitée 16 heures à température ambiante et le solide blanc formé est filtré, lavé à l'acétate d'éthyle puis à l'éther. Après recristallisation dans le méthanol, on obtient 3,7 g de produit (Rdt = 49 %).
F = 149°C, $[\alpha]_D^{20}$= -43,7 (c 1, eau).

| Analyse | C | H | N | Cl |
|---|---|---|---|---|
| Calc. | 38,09 | 5,32 | 11,84 | 7,49 |
| Tr. | 37,8 | 5,3 | 11,8 | 7,5 |

Exemple 62 : **5-[[[N-[(3-t-butoxycarbonyl-4-L-thiazolidinyl) carbonyl]-2-pyrrolidinyl (2S)] carbonyl] amino]-, 5-déoxy, 2-O-nitro, 1,4:3,6-dianhydro-L-iditol (69) :**

On agite à température ambiante une solution de 5,11 g (17,7 mmoles) de la forme base correspondant à (14) dans 200 ml de dichlorométhane anhydre. On ajoute successivement 4,12 g (17,7 mmoles) de N-t-Boc-L-thioproline, 3,65 g (17,7 mmoles) de DCC et 2,39 g (17,7 mmoles) d'HOBt. Après 24 heures de réaction et filtration du DCU, on ajoute 100 ml de dichlorométhane et on effectue les lavages comme pour (1). Le solide jaune obtenu après évaporation est chromatographié sur silice (230-400 mesh ASTM) et on isole 7,8 g d'une huile qui cristallise. Après recristallisation dans l'acétate d'éthyle, on obtient 5,4 g de produit (Rdt = 60 %).
F = 161-162°C, $[\alpha]_D^{20}$= -126,4 (c 1, éthanol).

| Analyse | C | H | N |
|---|---|---|---|
| Calc. | 47,80 | 6,02 | 11,15 |
| Tr. | 48,0 | 5,9 | 11,2 |

Exemple 63 : **5-[[[N-[(4-L-thiazolidinyl) carbonyl)-2-pyrrolidinyl (2S)] carbonyl] amino]-, 5-déoxy, 2-O-nitro, 1,4:3,6-dianhydro-L-iditol monochlorhydrate (70) :**

A 20 ml d'une solution d'acide chlorhydrique 2N dans l'acétate d'éthyle, on ajoute 2,86 g du composé précédent (69). La solution est agitée 18 heures à température ambiante et le solide blanc formé est filtré puis lavé à l'éther. Après recristallisation dans le méthanol, on obtient 2,06 g de produit (Rdt = 82 %).
F = 185°C (déc), $[\alpha]_D^{20}$= -133 (c 1, eau).

| Analyse | C | H | N | Cl |
|---|---|---|---|---|
| Calc. (0,1 $H_2O$ + 0,05 HCl) | 40,70 | 5,29 | 12,65 | 8,40 |
| Tr. | 40,7 | 5,3 | 12,3 | 8,4 |

Exemple 64 : **2-[[2-[[(4-L-thiazolidinyl) carbonyl] amino] 1-oxo éthyl] amino]-, 2-déoxy, 5-O-nitro, 1,4:3,6-dianhydro-D-glucitol, monochlorhydrate (71) :**

On agite à température ambiante une suspension de 5,3 g (21,4 mmoles) de la forme base correspondant à (16) dans 200 ml de dichlorométhane anhydre. On ajoute successivement 5 g (21,4 mmoles) de N-t-Boc-L-thioproline, 4,42 g (21,4 mmoles) de DCC et 2,9 g (21,4 mmoles) d'HOBt. Après 24 heures de réaction et filtration du DCU, on ajoute 100 ml de dichlorométhane et on effectue les lavages comme pour (1). Le résidu obtenu après évaporation est chromatographié sur silice (230-400 mesh ASTM) et on isole 5,75 g (Rdt = 58 %) d'une mousse sèche qui n'a pu être cristallisée. Le produit est traité directement.

A 41 ml d'une solution d'acide chlorhydrique 2N dans l'acétate d'éthyle, on ajoute 5,75 g du composé précédent. La solution est agitée 16 heures à température ambiante et le solide blanc formé est filtré, lavé à l'acétate d'éthyle puis à l'éther. Après recristallisation dans le méthanol, on obtient 2,6 g de produit (Rdt = 52 %).
F = 195°C (déc), $[\alpha]_D^{20}$= +24,5 (c 1, eau).

| Analyse | C | H | N | Cl |
|---|---|---|---|---|
| Calc. | 36,13 | 4,80 | 14,04 | 8,89 |
| Tr. | 36,0 | 4,8 | 14,0 | 9,1 |

Exemple 65 : **2-O-[2-[[(3-t-butoxycarbonyl-4-L-thiazolidinyl) carbonyl] amino] 1-oxo éthyl]-, 5-O-nitro, 1,4:3,6-dianhydro-D-glucitol (72) :**

On agite à température ambiante une suspension de 2,07 g (7, 27 mmoles) de (2) dans 60 ml de dichlorométhane anhydre. On ajoute 1,03 ml de triéthylamine (7,33 mmoles), ce qui provoque la dissolution du solide.

On ajoute successivement 1,7 g (7,27 mmoles) de N-t-Boc-L-thioproline, 3,08 g (7,27 mmoles) de CMC et 0,98 g (7,27 mmoles) d'HOBt. Après 24 heures de réaction, on ajoute 140 ml de dichlorométhane et on effectue les lavages comme pour (1). L'huile obtenue après évaporation est chromatographiée sur silice (230-400 mesh ASTM) et on isole 1,97 g d'un solide qui est recristallisé dans le mélange acétate d'éthyle-éther de pétrole. On obtient 1,71 g de produit (Rdt = 50 %).
F = 80-82°C, $[\alpha]_D^{20}$= -11,1 (c 1, éthanol).

| Analyse | C | H | N |
|---|---|---|---|
| Calc. | 44,06 | 5,44 | 9,07 |
| Tr. | 44,1 | 5,3 | 9,0 |

Exemple 66 : **2-O-[2-[[(4-L-thiazolidinyl) carbonyl] amino] 1-oxo éthyl]-, 5-O-nitro, 1,4:3,6-dianhydro-D-glucitol, monochlorhydrate (73) :**

On agite à température ambiante une suspension de 3,7 g (13 mmoles) de (2) dans 100 ml de dichlorométhane anhydre. On ajoute 1,83 ml de triéthylamine (13 mmoles), ce qui provoque la dissolution du solide. On ajoute successivement 3,03 g (22,75 mmoles) de -L-thioproline, 2,68 g (13 mmoles) de DCC et 1,76 g (13 mmoles) d'HOBt. Après 24 heures de réaction, on filtre le DCU et évapore le solvant. Le résidu est chromatographié sur silice (230-400 mesh ASTM) et on isole 4,1 g d'une huile. Celle-ci est diluée dans 80 ml de dichlorométhane et on fait barboter du chlorure d'hydrogène pendant quelques secondes. Le chlorhydrate précipite sous forme de pâte ; après élimination du solvant et lavage de la pâte au dichlorométhane puis à l'éther, on observe un durcissement du produit qui forme une poudre fine après filtration. Après triturations et lavages à l'hexane et à l'éther, on obtient finalement 2,84 g de produit (Rdt = 54 %).
F = 103°C, $[\alpha]_D^{20}$= +24,2 (c 1, eau).

| Analyse | C | H | N | Cl |
|---|---|---|---|---|
| Calc. (0,3 $H_2O$ + 0,05 HCl) | 35,41 | 4,61 | 10,32 | 9,14 |
| Tr. | 35,4 | 4,6 | 10,1 | 9,0 |

Exemple 67 : **5-O-[2-[[(4-L-thiazolidinyl) carbonyl] amino] 1-oxo éthyl]-, 2-O-nitro, 1,4:3,6-dianhydro-D-glucitol, monochlorhydrate (74) :**

On agite à température ambiante une suspension de 6,04 g (21,21 mmoles) de (3) dans 200 ml de dichlorométhane anhydre. On ajoute 2,98 ml de triéthylamine (21,21 mmoles), ce qui provoque la dissolution du solide. On ajoute successivement 4,95 g (21,21 mmoles) de N-t-Boc-L-thioproline, 8,99 g (21,21 mmoles) de CMC et 2,87 g (21,21 mmoles) d'HOBt. Après 24 heures de réaction, on ajoute 100 ml de dichlorométhane et on effectue les lavages comme pour (1). Le résidu obtenu après évaporation est chromatographié sur silice (230-400 mesh ASTM) et on isole 7,4 g d'une mousse sèche qui n'a pu être cristallisée. Le produit est traité directement.

A 62 ml d'une solution d'acide chlorhydrique 2N dans l'acétate d'éthyle, on ajoute 7,2 g du composé précédent. La solution est agitée 18 heures à température ambiante et après élimination du solvant, le résidu est lavé à l'acétate puis à l'éther. Après cristallisation et recristallisation dans l'éthanol, on obtient 2,7 g de produit (Rdt = 43 %).
F = 85°C, $[\alpha]_D^{20}$= +8,3 (c 1, eau).

| Analyse | C | H | N | Cl |
|---|---|---|---|---|
| Calc. (0,4 $H_2O$) | 35,41 | 4,65 | 10,32 | 8,71 |
| Tr. | 35,4 | 4,6 | 10,1 | 8,6 |

Exemple 68 : **5-[[2-[[(3-acétyl-4-L-thiazolidinyl) carbonyl] amino] 1-oxo éthyl] amino]-, 5-déoxy, 2-O-nitro, 1,4:3,6-dianhydro-L-iditol (75) :**

On agite à température ambiante une solution de 3 g (12,13 mmoles) de la forme base correspondant à (6) dans 120 ml de dichlorométhane anhydre. On ajoute successivement 2,13 g (12,13 mmoles) de N-acétyl-L-thioproline, 2,5 g (12,13 mmoles) de DCC et 1, 64 g (12,13 mmoles) d'HOBt. Après 24 heures de réaction et filtration du DCU, on ajoute 80 ml de dichlorométhane et on effectue les lavages comme pour (1). Le résidu huileux obtenu après évaporation est chromatographié sur silice (230-400 mesh ASTM). On isole un solide qui est recristallisé successivement dans l'acétate d'éthyle puis dans le méthanol. On obtient 2,2 g de produit (Rdt = 44 %).
F = 147-148°C, $[\alpha]_D^{20}$= -66,4 (c 1, eau).

| Analyse | C | H | N |
|---|---|---|---|
| Calc. | 41,58 | 4,99 | 13,85 |
| Tr. | 41,3 | 4,8 | 13,8 |

Exemple 69 : **5-[[2-[[(3-benzoyl-4-L-thiazolidinyl) carbonyl] amino] 1-oxo éthyl] amino]-, 5-déoxy, 2-O-nitro, 1,4:3,6-dianhydro-L-iditol (76) :**

On agite à température ambiante une solution de 1 g (4,05 mmoles) de la forme base correspondant à (6) dans 60 ml de dichlorométhane anhydre. On ajoute successivement 1,24 g (5,25 mmoles) de N-benzoyl-L-thioproline, 1,08 g (5,25 mmoles) de DCC et 0,71 g (5,25 mmoles) d'HOBt. Après 24 heures de réaction et filtration du DCU, on ajoute 40 ml de dichlorométhane et on effectue les lavages comme pour (1). On chromatographie le résidu obtenu après évaporation sur silice (230-400 mesh ASTM). On isole un solide blanc qui est recristallisé dans l'éthanol. On obtient 0,65 g de produit (Rdt = 26 %).
F = 135-136°C.

| Analyse | C | H | N |
|---|---|---|---|
| Calc. (0,2 $H_2O$) | 48,54 | 4,79 | 11,91 |
| Tr. | 48,5 | 4,7 | 11,8 |

Exemple 70 : **5-[[[[2-[(phénylméthyl)thio]benzoyl]amino]1-oxo éthyl]amino]-, 5-déoxy, 2-O-nitro, 1,4:3,6-dianhydro-L-iditol (47) :**

On agite à température ambiante une solution de 2 g (8,09 mmoles) de la forme base correspondant à (6) dans 70 ml de dichlorométhane anhydre. On ajoute successivement 2 g (8,19 mmoles) d'acide thiosalicylique S-benzylé et 1,68 g (8,19 mmoles) de DCC. Après 24 heures de réaction, filtration du DCU et évaporation du solvant, on chromatographie l'huile obtenue (silice 230-400 mesh ASTM) ; le composé isolé est recristallisé dans le méthanol. On obtient 2,08 g (Rdt = 54 %).
F = 113°C, $[\alpha]_D^{20}$= +22,5 (c 0,4 éthanol).

| Analyse | C | H | N |
|---|---|---|---|
| Calc. | 55,80 | 4,89 | 8,87 |
| Tr. | 55,6 | 4,9 | 8,8 |

**III-b : R-CO-A + Y-B :**

Le Tableau IV ci-après montre le dérivé synthétisé suivant cette méthode.

TABLEAU IV

| R-CO-A | Y-B |
|---|---|
| | 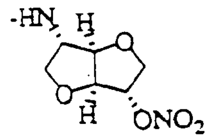 |
| 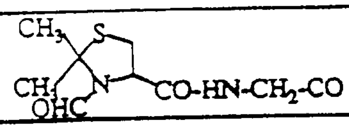 | 48 |

Exemple 71 : **5-[[2-[[(3-formyl-2,2-diméthyl-4-L-thiazolidinyl)carbonyl]amino]1-oxo éthyl]amino]-, 5-déoxy, 2-O-nitro, 1,4:3,6-dianhydro-L-iditol (48) :**

On agite à température ambiante une solution de 2,85 g (15 mmoles) de 1,4:3,6-dianhydro-5-déoxy-L-iditol-5-amino-2-nitrate dans 90 ml de dichlorométhane anhydre. On ajoute successivement 3,07 g (12,4 mmoles) de N-[4-(3-formyl-2,2 diméthyl-L-thiazolidine)carbonyl]-, glycine, 2,58 g (12,5 mmoles) de DCC et 1,68 g (12,4 mmoles) d'HOBt. Après 22 heures de réaction, on filtre le DCU et on évapore le solvant ; on chromatographie le résidu huileux sur silice (230-400 mesh ASTM) et on isole le composé qui est recristallisé dans le méthanol puis dans l'éthanol. On obtient 2,82 g (Rdt = 54 %).
F = 142-143°C, $[\alpha]_D^{20}$= +29,3 (c 2, acétone).

| Analyse | C | H | N |
|---|---|---|---|
| Calc. | 43,05 | 5,30 | 13,39 |
| Tr. | 43,2 | 5,2 | 13,4 |

**IV - SYNTHESE DES COMPOSES DE FORMULE I avec n = 0 et $R_2$= CO-X.**

| X \ R-CO-Y-B | 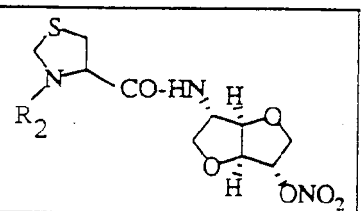 |
|---|---|
| 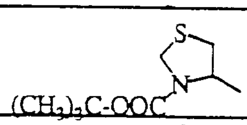 | 77 |
| 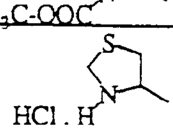 | 78 |

Exemple 72 : **5-[[[N-[(3-t-butoxycarbonyl-4-L-thiazolidinyl) carbonyl]-4-thiazolidinyl (4R)] carbonyl] amino]-, 5-déoxy, 2-O-nitro, 1,4:3,6-dianhydro-L-iditol (77) :**

On agite à température ambiante une solution de 4,65 g (15,2 mmoles) de la forme base correspondant à (30) dans 250 ml de dichlorométhane anhydre. On ajoute successivement 3,55 g (15,2 mmoles) de N-t-Boc-L-thioproline, 3,14 g (15,2 mmoles) de DCC et 2,06 g (15,2 mmoles) d'HOBt. Après 48 heures de réaction, on

filtre le DCU et évapore le solvant. Le résidu huileux est chromatographié sur silice (230-400 mesh ASTM) et on isole un solide blanc qui est recristallisé dans le mélange méthanol-éther diisopropylique, puis dans le méthanol. On obtient 3,2 g de produit (Rdt = 40 %).
F = 156°C, $[\alpha]_D^{20}$= -159,1 (c 1, éthanol).

| Analyse | C | H | N |
|---|---|---|---|
| Calc. | 43,84 | 5,42 | 10,76 |
| Tr. | 43,6 | 5,3 | 10,8 |

Exemple 73 : **5-[[[N-[(4-L-thiazolidinyl) carbonyl]-4-thiazolidinyl (4R)] carbonyl] amino]-, 5-déoxy, 2-O-nitro, 1,4:3,6-dianhydro-L-iditol monochlorhydrate (78) :**

A 12 ml d'une solution d'acide chlorhydride 2N dans l'acétate d'éthyle, on ajoute 1,8 g du composé précédent (77). La solution est agitée 18 heures à température ambiante et le solide blanc formé est filtré, lavé à l'acétate d'éthyle puis à l'éther. Après recristallisation dans le méthanol, on obtient 0,83 g de produit (Rdt = 52 %).
F = 174°C (déc), $[\alpha]_D^{20}$= -174,9 (c 1, eau).

| Analyse | C | H | N | Cl |
|---|---|---|---|---|
| Calc. | 36,80 | 4,63 | 12,26 | 7,76 |
| Tr. | 36,8 | 4,6 | 12,3 | 7,8 |

COMPTE RENDU PHARMACOLOGIQUE CONCERNANT LES COMPOSES SELON L'INVENTION.

**TESTS PHARMACOLOGIQUES :**

**Exemple A : Effet vasorelaxant des composés conformes à l'invention sur des vaisseaux isolés.**

Des rats Wistar adultes, pesant entre 250 et 350 g, sont anesthésiés par injection intrapéritonéale de pentobarbital sodique (30 mg/kg). L'aorte thoracique est prélevée rapidement et placée dans une solution de tampon bicarbonate de type Krebs-Ringer réfrigérée et oxygénée (composition [mM] : NaCl, 118 ; KCl, 4,7 ; $CaCl_2$, 2,5 ; $MgSO_4$, 1,2 ; $KH_2PO_4$, 1,2 ; $NaHCO_3$, 25,0 ; EDTA calcium disodique, 0,026 ; glucose, 11,1). L'aorte est disséquée dans le but d'enlever le tissu conjonctif, puis sectionnée en 6 anneaux (longueur 5-10 mm). L'enthothélium est ôté par abrasion modérée de la surface intimale des segments aortiques à l'aide d'une paire de pinces. Ces segments sont ensuite placés entre deux crochets dans une cuve à organes isolés remplie de 25 ml de tampon bicarbonate (pH 7,4) thermostaté à 37°C et oxygénée à l'aide d'un mélange 95 % $O_2$-5 % $CO_2$ (pH 7,4). Un des crochets est fixé à un point fixe, tandis que le second est raccordé à un transducteur, dans le but de mesurer les variations de tension isométrique (exprimées en grammes). Après une période d'équilibration de 30 min, les segments sont étirés progressivement de façon à atteindre le niveau de tension basale pour lequel la réponse contractile à la noradrénaline ($10^{-7}$ M) est maximale (tension optimale ; moyenne 2 g). L'absence d'endothélium est vérifiée par addition d'une concentration unique d'acétylcholine ($10^{-6}$ M), après pré-contraction des vaisseaux par la noradrénaline ($10^{-7}$ M). L'intégrité du muscle lisse vasculaire est également vérifiée en ajoutant une concentration unique d'un vasodilatateur dont la réponse est indépendante de la présence de l'endothélium, le SIN-1 ($10^{-5}$ M). Après cette procédure initiale, les aortes sont lavées, équilibrées pendant une période de 30 min, puis de nouveau contractées (noradrénaline $10^{-7}$ M). L'effet relaxant de la substance à tester est ensuite évalué en ajoutant des concentrations croissantes de cette substance (de $10^{-8}$ à $10^{-4}$ M). Le maximum d'inhibition de la contraction provoquée par la noradrénaline (exprimée en pourcentage) est ensuite calculé ("relaxation maximale"). Lorsque cette relaxation maximale est supérieure à 50 %, la concentration de substance testée produisant une inhibition de 50 % de la contraction maximale ($IC_{50}$) est calculée. Le Tableau V suivant rassemble les $IC_{50}$ et les pourcentages de relaxation maximale des différents composés testés.

TABLEAU V

| Numéro | $IC_{50}$ (µM) | Relaxation Maximale |
|---|---|---|
| 22 | 10 | 100 % |
| 30 | 10 | 100 % |
| 48 | > 100 | 25 % |
| 28 | 50 | 70 % |
| 39 | 50 | 80 % |
| 6 | > 100 | 45 % |
| 4 | 20 | 75 % |
| 5 | 25 | 70 % |
| 46 | 100 | 50 % |
| 32 | 15 | 100 % |
| 33 | > 100 | 50 % |
| 34 | > 100 | 25 % |
| 20 | 28 | 85 % |
| 31 | 25 | 83 % |
| 40 | > 100 | 38 % |
| 41 | 63 | 70 % |
| 42 | 33 | 92 % |
| 15 | 60 | 58 % |
| 16 | > 100 | 41 % |
| 1 | 32 | 83 % |
| 8 | 32 | 80 % |
| 37 | 13 | 100 % |
| 2 | 100 | 52 % |
| 14 | 14 | 98 % |
| 7 | 17 | 96 % |
| 13 | 49 | 74 % |
| 3 | 79 | 63 % |
| 11 | 25 | 100 % |
| 35 | 100 | 46 % |
| 26 | 80 | 70 % |
| 27 | > 100 | 14 % |
| 9 | 80 | 70 % |
| 18 | 72 | 75 % |
| 19 | > 100 | 40 % |
| 24 | 20 | 100 % |
| 25 | > 100 | 35 % |
| 43 | 32 | 80 % |
| 12 | 2,5 | 100 % |
| 21 | 100 | 31 % |
| 63 | 70 | 70 % |
| 60 | 87 | 53 % |
| 67 | 9 | 100 % |
| 49 | 38 | 100 % |
| 61 | 32 | 100 % |
| 77 | 40 | 97 % |

| Numéro | $IC_{50}$ (µM) | Relaxation Maximale |
|---|---|---|
| 69 | 42 | 87 % |
| 65 | 20 | 100 % |
| 62 | 55 | 72 % |
| 64 | 51 | 84 % |
| 70 | 35 | 83 % |
| 68 | 29 | 90 % |
| 72 | 64 | 67 % |
| 73 | 53 | 66 % |
| 71 | 70 | 66 % |
| 78 | 20 | 89 % |

D'une façon générale, les produits qui présentent une relaxation maximale supérieure à 80 % avec une $IC_{50}$ inférieure à 50 µM sont considérés comme particulièrement intéressants ; toutefois, ce Tableau ne prend pas en compte les données concernant la tachyphylaxie, et certains produits tel que le (46) peuvent, si l'on tient compte de ce critère supplémentaire, présenter un intérêt.

**Exemple B : Effets hémodynamiques des composés conformes à l'invention chez le chien éveillé hypertendu (n = 3 ).**

Le Tableau VI suivant et la figure 1 montrent plus particulièrement l'évolution en fonction du temps de l'effet du composé (46) sur les divers paramètres hémodynamiques, le composé étant administré p.o. à la dose de 3 mg/kg. Le composé (46) diminue de manière significative, prononcée et prolongée la pression artérielle systolique (PAs) et la pression télédiastolique du ventricule gauche (PTDVG). Les figures 1 (figures 1a, 1b, 1c, 1d, 1e) illustrent ces résultats et montrent les variations (4) des paramètres hémodynamiques suivants : PAs (figure 1a), PAd (figure 1b), PTDVG (figure 1c), FC (figure 1d) et activité vasorelaxante (figure 1e).

TABLEAU VI

A. Paramètres :

| | TEMPS (min) vb | 0 | 10 | 20 | 30 | 60 | 90 | 120 | 180 | 240 | 300 | 360 |
|---|---|---|---|---|---|---|---|---|---|---|---|---|
| PAs | 150 | 0 | -5 | 0 | 0 | -25 | -30 | -30 | -15 | -15 | -15 | -10 |
| | 185 | 0 | 0 | 0 | 0 | -15 | -25 | -25 | -25 | -35 | -25 | -25 |
| | 155 | 0 | -10 | -5 | -5 | -25 | -30 | -30 | -35 | -40 | -40 | -35 |
| PAd | 75 | 0 | -5 | 0 | 5 | 5 | 5 | 5 | 10 | 10 | 10 | 10 |
| | 85 | 0 | 0 | 0 | 0 | 0 | 10 | 10 | 15 | 15 | 15 | 15 |
| | 70 | 0 | -5 | 0 | 0 | -5 | -5 | 0 | 0 | 0 | 0 | 0 |
| PTDVG | 17 | 0 | -1 | -3 | 0 | -7 | -7 | -9 | -7 | -7 | -4 | -5 |
| | 13 | 0 | 0 | 0 | 0 | -3 | -6 | -7 | -4 | -5 | 0 | -3 |
| | 9 | 0 | -2 | -2 | -3 | -3 | -3 | -2 | -4 | -4 | -3 | -4 |
| dp/dt | 2500 | 0 | -100 | 300 | 0 | 0 | -200 | -400 | -300 | -500 | -300 | -500 |
| | 2300 | 0 | 0 | 0 | 500 | 500 | 500 | 800 | 500 | 500 | 800 | 500 |
| | 2400 | 0 | -400 | -200 | -200 | -400 | -400 | -400 | -200 | -400 | -400 | -400 |
| FC | 80 | 0 | 0 | 35 | 0 | 10 | 35 | 55 | 35 | 40 | 45 | 15 |
| | 95 | 0 | 10 | 0 | 10 | -10 | 45 | 65 | 40 | 20 | 40 | 10 |
| | 115 | 0 | 0 | 15 | 10 | -20 | -10 | -20 | 20 | 20 | -5 | 15 |

B. Moyennes : (n = 3)

| | TEMPS (min) vb | 0 | 10 | 20 | 30 | 60 | 90 | 120 | 180 | 240 | 300 | 360 |
|---|---|---|---|---|---|---|---|---|---|---|---|---|
| PAs moyenne | 163 | 0 | -5 | -2 | -2 | -22 | -28 | -28 | -25 | -30 | -27 | -23 |
| PAs SEM | 11 | 0 | 3 | 2 | 2 | 3 | 2 | 2 | 6 | 8 | 7 | 7 |
| PAd moyenne | 77 | 0 | -3 | 0 | 2 | 0 | 3 | 5 | 8 | 8 | 8 | 8 |
| PAd SEM | 4 | 0 | 2 | 0 | 2 | 3 | 4 | 3 | 4 | 4 | 4 | 4 |
| PTDVG moyenne | 13 | 0 | -1 | -2 | -1 | -4 | -5 | -6 | -5 | -5 | -2 | -4 |
| PTDVG SEM | 2 | 0 | 1 | 1 | 1 | 1 | 1 | 2 | 1 | 1 | 1 | 1 |
| dp/dt moyenne | 2400 | 0 | -167 | 33 | 100 | 33 | -33 | 0 | 0 | -133 | 33 | -133 |
| dp/dt SEM | 58 | 0 | 120 | 145 | 208 | 260 | 273 | 400 | 252 | 318 | 384 | 318 |
| FC moyenne | 97 | 0 | 3 | 17 | 7 | -7 | 23 | 33 | 32 | 27 | 27 | 13 |
| FC SEM | 10 | 0 | 3 | 10 | 3 | 9 | 17 | 27 | 6 | 7 | 16 | 2 |

PAs = pression artérielle systolique

PAd = pression artérielle diastolique

PTDVG = pression télédiastolique du ventricule gauche

dp/dt = activité vasorelaxante

FC = fréquence cardiaque

vb = valeur basale

**Exemple C : Effets hémodynamiques des composés conformes à l'invention chez le porc ou le chien anesthésié.**

Les porcs sont de race "German land" (20-26 kg) et sont utilisés après un jeûne d'une nuit.

Les chiens, pesant entre 16 et 26 kg, sont les descendants d'un croisement de Labrador et de Harrier.

Pour les porcs, la sédation, l'analgésie et l'anesthésie sont induites par la kétamine HCl (20 mg/kg i.m.), le méthonidate HCl (10 mg/kg i.m.), la xylacine HCl (3 mg/kg i.m.) et du pentobarbital sodique (25-30 mg/kg en bolus i.v. ou 0,16 mg/kg/min en perfusion i.v). Pour les chiens, l'anesthésie est induite à l'aide de pentobarbital sodique (45 mg/kg i.v.) et maintenue avec une perfusion i.v. (débit 8 mg/kg/h).

Les porcs ou les chiens sont mis sous respiration artificielle à l'air ambiant. L'analyse des gaz du sang ($pO_2$, $pCO_2$) est réalisée à intervalles de temps réguliers et de l'oxygène peut être délivré via le respirateur, si nécessaire.

Les paramètres hémodynamiques enregistrés incluent la pression ventriculaire gauche (PVG ; cathéter Millar PC350 Tip inséré via l'artère carotidienne), la pression sanguine systémique (PAs, PAd ; cathéter plein dans une artère fémorale), la pression de fin de diastole ventriculaire gauche (PTDVG), la contractilité ventriculaire ($PVGdp/dt_{max}$) et la fréquence cardiaque (FC).

Après une période d'équilibration de l'ordre de 45 minutes, les composés étudiés sont administrés par voie intraduodénale (i.d.) ou par voie intraveineuse (i.v.) chez plusieurs animaux (1 à 4). Les variables hémodynamiques sont enregistrées de manière continue pendant 1 à 3 heures, en fonction de la durée d'action des composés.

Les effets des composés étudiés sont présentés comme la différence absolue par rapport à la valeur basale (vb) mesurée avant l'administration des composés et sont présentés sur le Tableau VII (a, b, c).

Une activité antiangineuse s'apprécie par une diminution des PAs et des PTDVG (baisse du retour veineux).

TABLEAU VII a (i.d.)

| Effets hémodynamiques chez le Porc. | | | | | |
|---|---|---|---|---|---|
| Composé | Dose | PAs | | PTDVG | |
| | (mg/kg i.d.) | (mmHg) | (min) | (mmHg) | (min) |
| 6 | 1,0 | -31 | > 180 | -4,0 | > 180 |
| 5 | 1,0 | -24 | > 120 | -4,0 | > 120 |
| 30 | 3,0 | -40 | > 60 | -2,0 | > 60 |
| 28 | 3,0 | -28 | > 180 | -6,0 | > 180 |
| 4 | 3,0 | -28 | > 120 | -2,0 | > 120 |
| 46 | 3,0 | -30 | > 120 | -5,0 | > 120 |
| 32 | 3,0 | -30 | > 180 | -4,0 | > 180 |
| 48 | 10,0 | -25 | > 180 | -4,0 | > 180 |
| 39 | 10,0 | - 5 | > 90 | -1,0 | > 90 |
| 47 | 10,0 | -10 | > 120 | -1,0 | > 90 |
| 33 | 10,0 | -12 | > 60 | -3,0 | > 60 |
| 34 | 10,0 | -22 | > 180 | -4,0 | > 180 |
| 23 | 10,0 | 0 | | 0 | |
| 31 | 10,0 | -15 | > 60 | -2,0 | > 60 |
| IS-5-MN | 1,0 | 0 | | 0 | |
| IS-5-MN | 3,0 | - 5 | 60 | -3 | 60 |
| IS-5-MN | 10,0 | -26 | > 120 | -3,0 | > 120 |

TABLEAU VII b (i.V.)

| Effets hémodynamiques chez le Porc. | | | | | |
|---|---|---|---|---|---|
| Composé | Dose | PAs | | PTDVG | |
| | (mg/kg i.v.). | (mmHg) | (min) | (mmHg) | (min) |
| 29 | 3 | -42 | 10 | -2,0 | 16 |
| ? | 3 | -10 | 5 | 0,0 | 0 |
| 22 | 10 | -73 | 5 | -2,0 | 5 |
| 20 | 10 | -20 | 5 | -4,0 | 5 |

TABLEAU VII c (i.d.)

| Effets hémodynamiques chez le Chien. | | | | | |
|---|---|---|---|---|---|
| Composé | Dose | PAs | | PTDVG | |
| | (mg/kg i.d.) | (mmHg) | (min) | (mmHg) | (min) |
| 68 | 1 | -30 | > 180 | -3,0 | 180 |
| 49 | 1,1 | -32 | > 240 | | |
| 41 | 3 | -25 | > 180 | 0,0 | 0 |
| 15 | 3 | -20 | > 180 | -3,0 | > 180 |
| 8 | 3 | -30 | > 150 | | |
| 13 | 3 | -30 | > 120 | | |
| 3 | 3 | -10 | > 60 | -4,5 | > 60 |
| 12 | 3 | -30 | > 240 | | |
| 21 | 3 | -5 | > 60 | -2,0 | > 60 |
| 9 | 3 | 5 | > 60 | 0,0 | 0 |
| 60 | 3 | -15 | > 60 | 0,0 | 0 |
| 67 | 3 | -15 | > 90 | -1,5 | > 90 |
| 61 | 3 | -25 | > 180 | -1,5 | > 180 |
| 77 | 3 | -35 | > 180 | -2,0 | > 180 |
| 69 | 3 | -40 | > 150 | -2,5 | > 150 |
| 65 | 3 | 0 | 0 | 1,0 | 30 |
| 62 | 3 | -35 | > 30 | | |
| 64 | 3 | -25 | > 180 | -2,0 | 150 |
| 70 | 3 | -30 | > 90 | 0,0 | 0 |
| 71 | 3 | 0 | 0 | -5,5 | > 60 |
| 78 | 3 | -13 | 150 | -1,0 | 90 |
| 25 | 10 | -30 | > 60 | -3,0 | > 60 |

TABLEAU VII d (i.v.)

| Effets hémodynamiques chez le Chien. | | | | | |
|---|---|---|---|---|---|
| Composé | Dose | PAs | | PTDVG | |
| | (mg/kg i.v.) | (mmHg) | (min) | (mmHg) | (min) |
| 45 | 3 | -10 | > 120 | 0,0 | 0 |
| 40 | 3 | -10 | > 90 | -1,0 | > 90 |
| 1 | 3 | 5 | 10 | 1,0 | 10 |
| 2 | 3 | -10 | > 120 | -1,0 | > 120 |
| 14 | 3 | -10 | > 90 | -2,0 | > 90 |
| 7 | 3 | -20 | > 90 | -2,0 | > 90 |
| 11 | 3 | -10 | > 30 | 0,0 | 0 |
| 35 | 3 | -10 | > 30 | 0,0 | 0 |
| 27 | 3 | -15 | > 90 | -1,0 | > 90 |
| 63 | 3 | -20 | > 60 | -1,0 | 60 |
| 24 | 3 | -10 | > 30 | 1,0 | 10 |
| 73 | 3 | -15 | 60 | -2,0 | 60 |
| 42 | 10 | -10 | > 90 | 0,0 | 0 |
| 16 | 10 | -5 | > 30 | -1,0 | > 30 |
| 37 | 10 | -25 | > 90 | -2,0 | 30 |
| 26 | 10 | -10 | > 90 | 2,0 | > 90 |
| 18 | 10 | -10 | > 30 | 3,0 | > 30 |
| 19 | 10 | -35 | > 60 | 0,0 | 0 |
| 43 | 10 | -20 | 90 | -1,0 | 60 |
| 72 | 10 | 0 | 0 | 0,0 | 0 |

**Exemple D : Mesure de l'activité relaxante des composés conformes à l'invention sur les vaisseaux sanguins isolés de cobaye.**

L'artère pulmonaire de cobayes mâles (350-500 g) est isolée et coupée en bandelettes spiralées (1 mm de large, 15 mm de long). Celles-ci sont disposées dans une cuve à organes à 37°C remplie d'une solution de NaCl à 0,9 % et oxygénée à l'aide d'un mélange 95 % $O_2$-5 % $CO_2$ (Tyrode, pH 7,4), pour mesurer les variations de longueur.

Après avoir enlevé le calcium des tissus (Tyrode sans calcium et EDTA 0,2 mM), les bandes sont dépolarisées en présence d'une solution concentrée en ions K+ (40 mM), qui est réalisée en remplaçant le NaCl par des quantités équimolaires de KCl, puis une contraction de longue durée est induite par des ions $Ca^{2+}$ (0,5 mM).

Les composés étudiés sont ensuite introduits dans la cuve à organes à des concentrations croissantes.

Chaque composé est testé sur au moins quatre bandelettes spiralées de vaisseau.

La puissance de relaxation des composés est mesurée en calculant les concentrations qui donnent une relaxation de 50 % ($IC_{50}$), à partir des courbes concentration/effet. Le Tableau VIII suivant illustre ces résultats.

TABLEAU VIII

| Effets relaxant sur le vaisseau pulmonaire isolé chez le cobaye. | |
|---|---|
| Composé | $IC_{50}$ (µM) |
| 6 | 30 |
| 5 | 14 |
| 30 | 32 |
| 28 | 2 |
| 4 | 6 |
| 46 | 38 |
| 48 | > 100 |
| 39 | 1 |
| 47 | 2 |
| 22 | 3 |
| IS-5-MN | > 100 |

L'$IC_{50}$ illustre l'effet relaxant sur le vaisseau pulmonaire isolé.

**Exemple E : Absence de tachyphylaxie chez le chien.**

**Comparaison avec le mononitrate d'isosorbide (IS-5-MN).**

Le produit (46) est testé dans un modèle de tachyphylaxie, chez le chien anesthésié. L'IS-5-MN est utilisé comme substance de référence.

8 chiens de chaque sexe, pesant entre 16 et 24 kg sont utilisés. Ces chiens sont les descendants d'un croisement de Labrador et Harrier. L'anesthésie est induite à l'aide de pentobarbital sodique (45 mg/kg i.v.) et maintenue avec une perfusion i.v. (débit : 8 mg/kg/h). Les animaux sont ventilés, à l'aide d'un respirateur de type "Bird Mark 7", utilisant l'air ambiant, et cathétérisés. La pression artérielle périphérique systémique (PAs) est mesurée dans une artère fémorale au moyen d'un transducteur de pressions (Combitrans, B. Braun Melsungen AG, 3508 Delsungen, Allemagne). Les gaz du sang artériel sont mesurés à intervalles réguliers.

Le composé (46) est administré par voie intraduodénale (i.d.) à la dose de 0,75 mg/kg et sous un volume de 10 ml/animal, dissous dans de l'eau. L'IS-5-MN est également dissous dans l'eau et donné par voie i.d. à la dose de 5 mg/kg dans un volume de 10 ml/animal.

Trois heures (dans le cas de l'IS-5-MN) ou quatre heures (dans le cas du (46)) après la première administration, les composés sont réadministrés à la même dose et par la même voie. Le paramètre hémodynamique choisi pour l'étude de la tachyphylaxie est la pression artérielle systolique (PAs), dans la mesure où ce paramètre est très sensible à l'administration des composés.

Au moment de la seconde administration des composés conformes à l'invention, la PAs est revenue approximativement à la valeur contrôle (avant la première administration).

Les résultats obtenus sont présentés dans les Tableaux IX et X, qui correspondent aux figures 2 [2a (témoin : IS-5-MN) et 2b (composé (46))] qui illustrent l'effet en fonction du temps d'une administration réitérée de ces produits sur la PAs chez le chien anesthésié ; les courbes (-•-) correspondent à la première administration et les courbes (...○...) correspondent à la deuxième administration.

TABLEAU IX.A : 1ère Administration de IS-5-MN

1. Paramètres :

| | TEMPS (min) | | | | | | | | |
|---|---|---|---|---|---|---|---|---|---|
| | vb | 0 | 5 | 10 | 15 | 30 | 60 | 120 | 180 |
| PAs | 140 | 0 | -25 | -25 | -25 | -35 | -35 | 0 | -5 |
| | 135 | 0 | -35 | -25 | -20 | -20 | -10 | 0 | 0 |
| | 160 | 0 | -30 | -30 | -30 | -25 | -25 | -15 | -15 |
| | 135 | 0 | -30 | -25 | -25 | -15 | -20 | - 5 | 5 |
| PAd | 90 | 0 | -15 | -15 | -15 | -15 | -20 | 10 | 5 |
| | 90 | 0 | -25 | -15 | -10 | -10 | 0 | 0 | 0 |
| | 110 | 0 | -20 | -20 | -20 | -15 | -15 | -5 | -5 |
| | 90 | 0 | -25 | -20 | -20 | -10 | -15 | 5 | 10 |
| PTDVG | 15 | 0 | -3 | -3 | -3 | -1 | 0 | -1 | -3 |
| | 10 | 0 | -4 | -5 | -5 | -5 | -2 | -1 | -1 |
| | 13 | 0 | -3 | -3 | -3 | -3 | -3 | -3 | -5 |
| | 12 | 0 | -2 | -1 | -1 | -1 | -1 | -2 | 0 |
| dp/dt | 1400 | 0 | -200 | -300 | -200 | -300 | -400 | 400 | 100 |
| | 1600 | 0 | -100 | 0 | 0 | 0 | 0 | 0 | 100 |
| | 2000 | 0 | -100 | -100 | -100 | -100 | -100 | 0 | 100 |
| | 2100 | 0 | -300 | -300 | -300 | -300 | -300 | -100 | 100 |
| FC | 140 | 0 | 0 | 0 | -5 | -15 | -20 | 0 | -5 |
| | 125 | 0 | 0 | 5 | 5 | 5 | 10 | 10 | 10 |
| | 130 | 0 | 5 | 5 | 5 | 0 | -5 | 0 | 5 |
| | 140 | 0 | 0 | 0 | 0 | 0 | 0 | 5 | 10 |

2. Moyennes :

| | TEMPS (min) | | | | | | | | |
|---|---|---|---|---|---|---|---|---|---|
| | vb | 0 | 5 | 10 | 15 | 30 | 60 | 120 | 180 |
| PAs moyenne | 143 | 0 | -30 | -26 | -25 | -24 | -23 | -5 | -4 |
| SEM | 6 | 0 | 2 | 1 | 2 | 4 | 5 | 4 | 4 |
| PAd moyenne | 95 | 0 | -21 | -18 | -16 | -13 | -12 | 3 | 3 |
| SEM | 5 | 0 | 2 | 1 | 2 | 1 | 4 | 3 | 3 |
| PTDVG moyenne | 13 | 0 | -3 | -3 | -3 | -3 | -1 | -2 | -2 |
| SEM | 1 | 0 | 0 | 1 | 1 | 1 | 1 | 0 | 1 |
| dp/dt moyenne | 1775 | 0 | -175 | -175 | -150 | -175 | -200 | 75 | 100 |
| SEM | 165 | 0 | 48 | 75 | 65 | 75 | 91 | 111 | 0 |
| FC moyenne | 134 | 0 | 1 | 3 | 1 | -2 | -4 | 4 | 5 |
| SEM | 4 | 0 | 1 | 1 | 2 | 4 | 6 | 2 | 4 |

TABLEAU IX.B : 2ème Administration de IS-5-MN

1. Paramètres :

| | TEMPS (min) vb | 0 | 5 | 10 | 15 | 30 | 60 | 120 | 180 |
|---|---|---|---|---|---|---|---|---|---|
| PAs | 135 | 0 | -15 | -15 | -15 | -10 | 0 | -5 | -5 |
| | 135 | 0 | -20 | -15 | -15 | -10 | -10 | -15 | -5 |
| | 145 | 0 | -20 | -15 | -10 | -5 | -15 | -5 | 0 |
| | 140 | 0 | -20 | -15 | -10 | -10 | -5 | 0 | -5 |
| PAd | 90 | 0 | -5 | -5 | -5 | 0 | 5 | 0 | 0 |
| | 95 | 0 | -15 | -15 | -15 | -10 | -10 | -15 | -5 |
| | 105 | 0 | -15 | -15 | -10 | -5 | -10 | -5 | 0 |
| | 100 | 0 | -15 | -10 | -5 | -5 | 0 | 0 | 0 |
| PTDVG | 12 | 0 | -2 | -2 | -2 | -2 | -2 | -2 | -2 |
| | 9 | 0 | -2 | -2 | -1 | -1 | -3 | -2 | -3 |
| | 8 | 0 | 0 | -1 | 0 | 0 | -1 | 1 | -1 |
| | 12 | 0 | -2 | -2 | -2 | -2 | -1 | -2 | -2 |
| dp/dt | 1400 | 0 | -200 | -200 | -100 | 0 | 100 | 0 | 100 |
| | 1700 | 0 | -100 | 0 | -100 | 0 | 0 | 0 | 200 |
| | 2100 | 0 | -100 | -100 | -100 | 0 | -100 | 100 | 300 |
| | 2200 | 0 | -100 | 0 | -100 | 0 | 200 | 300 | 100 |
| FC | 135 | 0 | 0 | 0 | -5 | 0 | 5 | 5 | 5 |
| | 135 | 0 | 0 | 5 | 0 | 5 | 0 | 5 | 15 |
| | 135 | 0 | 0 | 5 | 5 | 0 | -5 | -5 | 0 |
| | 150 | 0 | 5 | 5 | 0 | 0 | 0 | 10 | 5 |

2. Moyennes : (n = 4)

| | TEMPS (min) vb | 0 | 5 | 10 | 15 | 30 | 60 | 120 | 180 |
|---|---|---|---|---|---|---|---|---|---|
| PAs moyenne | 139 | 0 | -19 | -15 | -13 | -9 | -7 | -5 | -4 |
| SEM | 2 | 0 | 1 | 0 | 1 | 1 | 3 | 3 | 1 |
| PAd moyenne | 98 | 0 | -13 | -11 | -9 | -5 | -4 | -5 | -1 |
| SEM | 3 | 0 | 3 | 2 | 2 | 2 | 4 | 4 | 1 |
| PTDVG moyenne | 10 | 0 | -1 | -2 | -1 | -1 | -2 | -1 | -2 |
| SEM | 1 | 0 | 1 | 0 | 1 | 1 | 0 | 1 | 0 |
| dp/dt moyenne | 1850 | 0 | -125 | -75 | -100 | 0 | 50 | 100 | 175 |
| SEM | 185 | 0 | 25 | 48 | 0 | 0 | 65 | 71 | 48 |
| FC moyenne | 139 | 0 | 1 | 4 | 0 | 1 | 0 | 4 | 6 |
| SEM | 4 | 0 | 1 | 1 | 2 | 1 | 2 | 3 | 3 |

TABLEAU X.A : 1ère Administration du composé (46)

1. Paramètres :

| | TEMPS (min) | | | | | | | | |
|---|---|---|---|---|---|---|---|---|---|
| | vb | 5 | 10 | 15 | 30 | 60 | 120 | 180 | 240 |
| PAs | 130<br>145<br>120<br>145 | -5<br>-5<br>-10<br>0 | -15<br>-20<br>-20<br>-15 | -25<br>-25<br>-20<br>-35 | -30<br>-30<br>-20<br>-40 | -30<br>-35<br>-15<br>-50 | -30<br>-35<br>-15<br>-45 | -25<br>-30<br>-10<br>-30 | -15<br>-20<br>0<br>-15 |
| PAd | 80<br>95<br>80<br>90 | -5<br>-5<br>-5<br>0 | -10<br>-10<br>-10<br>-15 | -15<br>-15<br>-15<br>-20 | -20<br>-20<br>-15<br>-25 | -20<br>-25<br>-10<br>-25 | -20<br>-25<br>-10<br>-25 | -20<br>-20<br>-5<br>-15 | -10<br>-20<br>0<br>0 |
| PTDVG | 9<br>10<br>10<br>13 | 0<br>-1<br>0<br>0 | -2<br>-2<br>0<br>-3 | -3<br>-2<br>1<br>-3 | -4<br>-2<br>1<br>-4 | -3<br>-3<br>-2<br>-4 | -2<br>-2<br>-6<br>-4 | -3<br>-2<br>-7<br>-4 | -4<br>-3<br>-7<br>-3 |
| dp/dt | 1100<br>1300<br>1200<br>1600 | 0<br>0<br>0<br>0 | -100<br>-100<br>-100<br>-100 | -100<br>-100<br>-100<br>-200 | -100<br>-200<br>-100<br>-200 | -200<br>-300<br>0<br>-300 | -200<br>-300<br>-200<br>-300 | -200<br>-300<br>-100<br>0 | -100<br>-100<br>0<br>300 |
| FC | 105<br>125<br>125<br>115 | 0<br>-5<br>0<br>0 | 0<br>-5<br>0<br>0 | 0<br>-5<br>0<br>0 | -5<br>-5<br>0<br>0 | -10<br>-10<br>-5<br>-5 | -15<br>-15<br>-15<br>5 | -10<br>-15<br>-10<br>10 | -5<br>-10<br>0<br>15 |

2. Moyennes : (n = 4)

| | TEMPS (min) | | | | | | | | |
|---|---|---|---|---|---|---|---|---|---|
| | vb | 5 | 10 | 15 | 30 | 60 | 120 | 180 | 240 |
| PAs<br>moyenne<br>SEM | 135<br>6 | -5<br>2 | -18<br>1 | -26<br>3 | -30<br>4 | -33<br>7 | -31<br>6 | -24<br>5 | -12<br>4 |
| PAd<br>moyenne<br>SEM | 86<br>4 | -4<br>1 | -11<br>1 | -16<br>1 | -20<br>2 | -20<br>4 | -20<br>4 | -15<br>4 | -7<br>5 |
| PTDVG<br>moyenne<br>SEM | 11<br>1 | -0<br>0 | -2<br>1 | -2<br>1 | -2<br>1 | -3<br>0 | -4<br>1 | -4<br>1 | -4<br>1 |
| dp/dt<br>moyenne<br>SEM | 1300<br>108 | 0<br>0 | -100<br>0 | -125<br>25 | -150<br>29 | -200<br>71 | -250<br>29 | -150<br>65 | 25<br>95 |
| FC<br>moyenne<br>SEM | 118<br>5 | -1<br>1 | -1<br>1 | -1<br>1 | -2<br>1 | -8<br>1 | -10<br>5 | -6<br>6 | 0<br>5 |

73

TABLEAU X.B : 2ème Administration du composé (46)

1. Paramètres :

| | TEMPS (min) | | | | | | |
|---|---|---|---|---|---|---|---|
| | vb | 5 | 10 | 15 | 30 | 60 | 120 |
| PAs | 115<br>125<br>120<br>140 | -5<br>-10<br>-5<br>-5 | -15<br>-15<br>-5<br>-10 | -20<br>-20<br>-15<br>-15 | -25<br>-20<br>-20<br>-35 | -30<br>-30<br>-20<br>-50 | -20<br>-30<br>-20<br>-35 |
| PAd | 70<br>75<br>80<br>95 | -5<br>-5<br>-5<br>-5 | -10<br>-5<br>-5<br>-5 | -15<br>-10<br>-5<br>-10 | -15<br>-10<br>-10<br>-25 | -15<br>-15<br>-10<br>-35 | -10<br>-15<br>-10<br>-25 |
| PTDVG | 5<br>7<br>3<br>10 | 0<br>0<br>1<br>0 | 0<br>0<br>0<br>0 | -1<br>-1<br>2<br>0 | -1<br>-1<br>0<br>-2 | -1<br>-1<br>-1<br>-3 | -1<br>-1<br>-2<br>-3 |
| dp/dt | 1000<br>1200<br>1200<br>2200 | 0<br>0<br>-100<br>-100 | 0<br>0<br>-100<br>-200 | -100<br>-100<br>0<br>-300 | -100<br>-100<br>-100<br>-600 | -100<br>-200<br>-200<br>-900 | -100<br>-200<br>-200<br>-600 |
| FC | 100<br>115<br>125<br>130 | 0<br>0<br>0<br>0 | 0<br>0<br>0<br>0 | -5<br>0<br>0<br>0 | 0<br>-5<br>0<br>0 | -5<br>-10<br>0<br>0 | 0<br>-15<br>0<br>5 |

2. Moyennes : (n = 4)

| | TEMPS (min) | | | | | | |
|---|---|---|---|---|---|---|---|
| | vb | 5 | 10 | 15 | 30 | 60 | 120 |
| PAs<br>moyenne<br>SEM | 125<br>5 | -6<br>1 | -11<br>2 | -18<br>1 | -25<br>4 | -33<br>6 | -26<br>4 |
| PAd<br>moyenne<br>SEM | 80<br>5 | -5<br>0 | -6<br>1 | -10<br>2 | -15<br>4 | -19<br>6 | -15<br>4 |
| PTDVG<br>moyenne<br>SEM | 6<br>1 | 0<br>0 | 0<br>0 | 0<br>1 | -1<br>0 | -2<br>1 | -2<br>0 |
| dp/dt<br>moyenne<br>SEM | 1400<br>271 | -50<br>29 | -75<br>48 | -125<br>63 | -225<br>125 | -350<br>185 | -275<br>111 |
| FC<br>moyenne<br>SEM | 118<br>7 | 0<br>0 | 0<br>0 | -1<br>1 | -1<br>1 | -4<br>2 | -2<br>4 |

A la première administration, l'IS-5-MN à 5 mg/kg et le composé (46) à 0,75 mg/kg induisent, en moyenne,

une chute similaire (-30 mm de Hg) de la PAs.

L'effet maximal est atteint 5 minutes après l'administration pour l'IS-5-MN et 60 min après pour le composé (46). La durée d'action du composé (46) est plus longue, raison pour laquelle l'intervalle est de 4 heures entre la première et la deuxième administration. La deuxième administration de IS-5-MN entraîne une chute de la PA qui atteint son maximum à 5 min (-19 mm de Hg) suivie d'un retour rapide à la valeur contrôle. La deuxième administration du composé (46) entraîne une chute de la PA, qui atteint son maximum à 60 min (-33 mm de Hg).

L'étude est interrompue 120 minutes après la deuxième administration.

Ces résultats confirment les données antérieures en ce qui concerne l'IS-5-MN (développement d'une tachyphylaxie après une administration répétée), chez le chien.

Ils montrent par ailleurs, l'absence de développement d'une tachyphylaxie après l'administration de (46).

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la-portée, de la présente invention.

## Revendications

**1°)** Nitrates organiques, caractérisés en ce qu'ils répondent à la formule I suivante :

$$R - CO - (A)_n - Y - B \quad (I)$$

dans laquelle :

**R représente** :

a. l'un des restes C, D, E, F et G suivants :

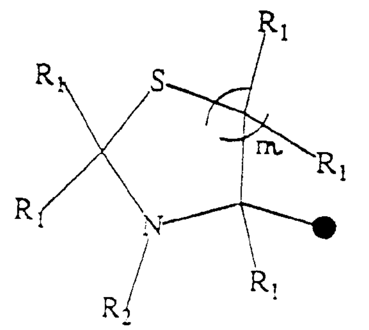

(reste C),

reste C dans lequel :

**$R_1$** représente un atome d'hydrogène, un groupe alkyle en $C_1$ à $C_6$ (linéaire, ramifié ou cyclique), un phényle éventuellement substitué ou un benzyle éventuellement substitué ;

**$R_2$** représente un atome d'hydrogène, un groupe alkyle en $C_1$ à $C_6$ (linéaire, ramifié ou cyclique), un phényle éventuellement substitué, un benzyle éventuellement substitué, un groupe acyle en $C_1$-$C_6$, un benzoyle éventuellement substitué, un alcoxy-carbonyle ou un groupe CO-X dans lequel X représente un reste C ou un reste D, E, F ou G tels que définis ci-après, et **m** représente 1 ou 2.

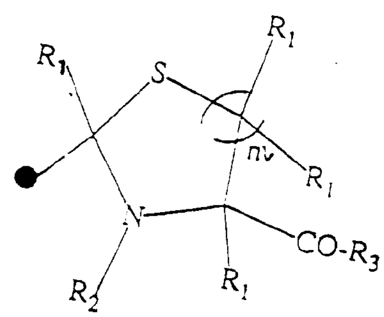

(reste D),

reste D dans lequel :

**$R_1$** et **$R_2$** ont la même signification que ci-dessus ;

**$R_3$** représente un groupe OH, un groupe O-alkyle en $C_1$ à $C_6$ (linéaire, ramifié ou cyclique), un groupe O-phényle éventuellement substitué, un groupe O-benzyle éventuellement substitué, un reste E tel que défini ci-après, un reste de type Y-B- ou un groupe NH-CH($COOR_3$)$CH_2$-S-$R_4$, dans lequel $R_4$ représente un atome d'hydrogène, un groupe alkyle en $C_1$ à $C_6$ (linéaire, ramifié ou cyclique), un phényle éventuellement substitué, un S-phényle éventuellement substitué, un benzyle éventuellement substitué, ou l'un des groupes suivants : $CH_3$-CO-NH-$CH_2$, B-Y-CO-CH(NH-COO-C($CH_3$)$_3$)$CH_2$-S, ou B-Y-CO-CH($NH_2$.HCl)$CH_2$-S, B et Y étant tels que définis ci-après, et **m** représente 1 ou 2.

- 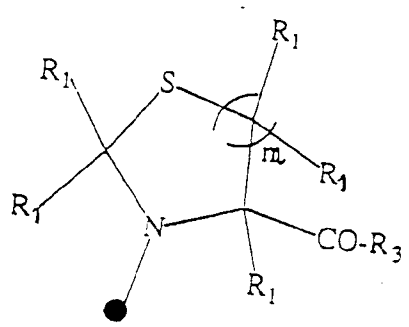 (reste E),

reste E dans lequel :
**$R_1$**, **$R_3$** et **m** ont la même signification que ci-dessus.

- 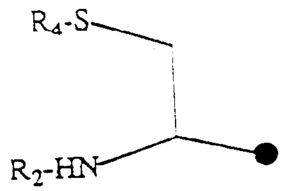 (reste F),

reste F dans lequel :
**$R_2$** et **$R_4$** ont la même signification que ci-dessus

- 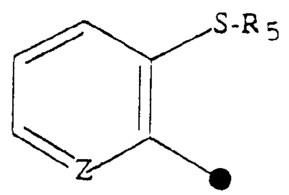 (reste G),

reste G dans lequel :
**$R_5$** représente un atome d'hydrogène, un groupe alkyle en $C_1$ à $C_6$ (linéaire, ramifié ou cyclique), un phényle éventuellement substitué, un S-phényle éventuellement substitué, un benzyle éventuellement substitué, un groupe $CH_3$-CO-NH-$CH_2$ ou B-Y-CO-$C_5ZH_3$ -S et **Z** représente CH ou N.
b. un résidu d'aminoacide soufré, éventuellement protégé.
c. R représente également, lorsque n est différent de 0 et A est un reste soufré H ou I tels que définis ci-après, un groupe OH, un groupe O-alkyle en $C_1$-$C_6$ (linéaire, ramifié ou cyclique), un groupe O-phényle éventuellement substitué, un groupe O-benzyle éventuellement substitué, un reste E tel que défini ci-dessus ou un reste Y-B, B et Y étant tels que définis ci-après ;

**A représente** un groupe $CH_2$, un acide aminé substitué ou non, avec la fonction acide liée à Y et la fonction amine liée à CO ou l'un des restes suivants :

– 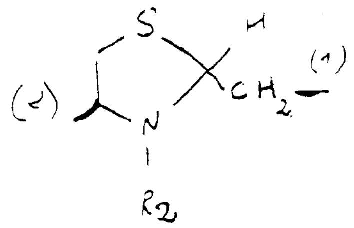 (reste H),

reste H dans lequel:

**$R_2$** représente un atome d'hydrogène, un groupe alkyle en $C_1$ à $C_6$ (linéaire, ramifié ou cyclique), un phényle éventuellement substitué, un benzyle éventuellement substitué, un groupe acyle en $C_1$ à $C_6$, un benzoyle éventuellement substitué, un groupe alcoxy-carbonyle, ou un groupe CO-X dans lequel X représente l'un des restes C, D, E, F ou G tels que définis ci-dessus pour R,

**(1)** est la liaison avec Y,

**(2)** est la liaison avec R-CO.

– 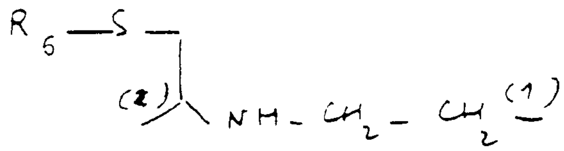 (reste I),

reste I dans lequel :

**$R_6$** représente un atome d'hydrogène, un groupe alkyle en $C_1$ à $C_6$ (linéaire, ramifié ou cyclique), un phényle éventuellement substitué, un S-phényle substitué, un benzyle éventuellement substitué ou l'un des groupes suivants : $CH_3$-CO-NH-$CH_2$, S-$CH_2$-CH-(CO-$R_7$)-NH-$CH_2$-$CH_2$-NH-B dans lequel $R_7$ représente un groupe OH, un groupe O-alkyle en $C_1$-$C_6$, un groupe O-phényle éventuellement substitué, un groupe O-benzyle éventuellement substitué, un reste E tel que défini ci-dessus ou un reste Y-B, B et Y étant tels que définis ci-après, **(1)** et **(2)** ont la même signification que ci-dessus.

**n est** égal à 0 ou 1 ou supérieur à 1 ;

**Y représente** un atome d'oxygène ou un groupe NH.

**B représente :**

α) un reste dianhydro 1,4:3,6 hexitol mono-nitrate de formule (a)

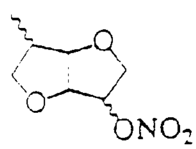

β) un reste itol nitrate en $C_1$ à $C_6$ de formules (b)

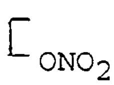 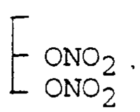 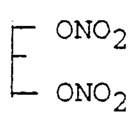 ........→ $C_6$

($b_1$) ($b_2$) ($b_3$) ....

γ) un reste d'inositol "p" nitrates, p étant un nombre entier de 1 à 5, de formule (c)

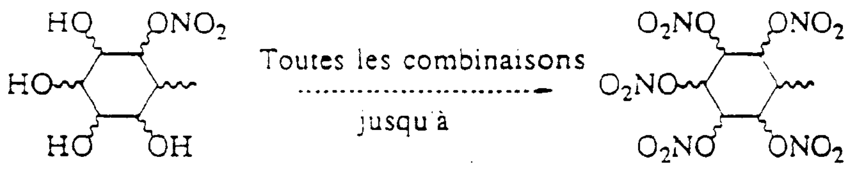


(c1) . . . . . . . . . . . . (c5)

δ) l'un des groupes suivants :

. un groupe $-CH_2-C(CH_2-ONO_2)_3$, dérivé du pentaérythritol,

. un groupe $-CH_2-C(C_2H_5)(CH_2-ONO_2)_2$, dérivé de l'éthyl-triméthylol-méthane,

. un groupe $-CH_2-CH_2-N(CH_2-CH_2-ONO_2)_2$, dérivé de la triéthanolamine,

avec toutes les combinaisons OH, $ONO_2$.

**2°)** Nitrates organiques selon la revendication 1, caractérisés en ce qu'ils comprennent pour R, A, Y et B, respectivement les radicaux suivants :

**R représente** l'un des restes suivants :

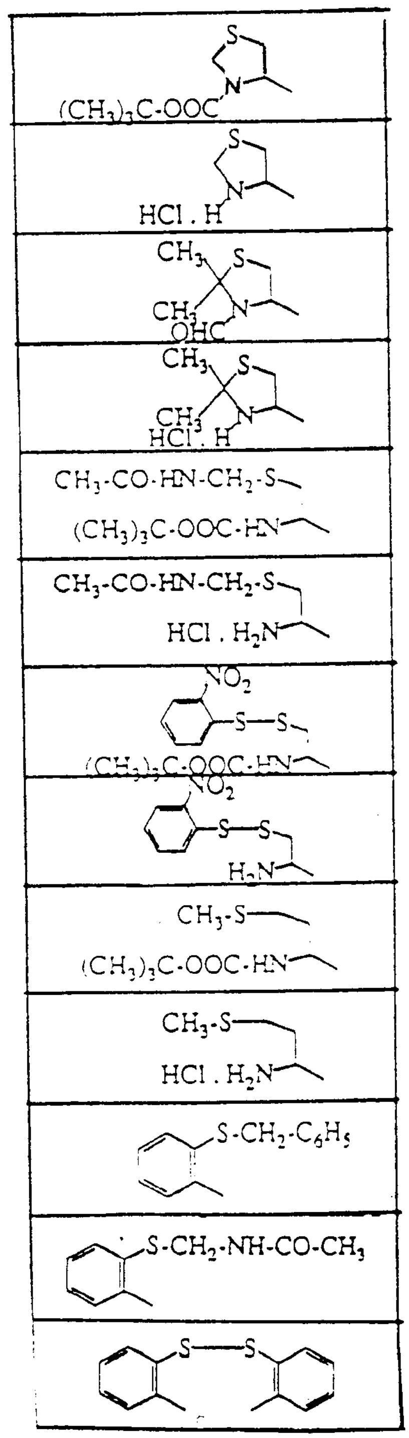

A représente un reste d'aminoacide éventuellement substitué, lorsque n est différent de 0, et notamment la glycine et ses dérivés, la proline et ses dérivés, l'alanine et ses dérivés, la valine et ses dérivés, et la phénylalanine et ses dérivés.

Y représente un atome d'oxygène ou un groupe NH,

B est un reste dérivé des composés suivants :

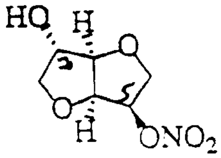
1,4:3,6-dianhydro-D-glucitol-5-nitrate

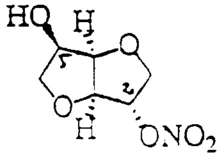
1,4:3,6-dianhydro-D-glucitol-2-nitrate

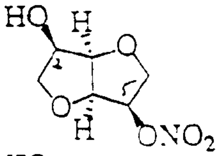
1,4:3,6-dianhydro-D-manitol-5-nitrate

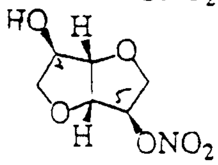
1,4:3,6-dianhydro-D-iditol-5-nitrate

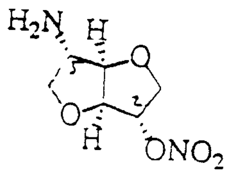
1,4:3,6-dianhydro-5-déoxy-L-iditol-5-amino-2-nitrate

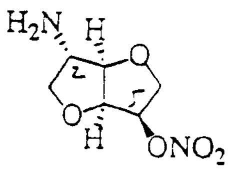
1,4:3,6-dianhydro-2-déoxy-D-glucitol-2-amino-5-nitrate

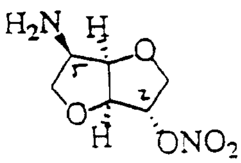
1,4:3,6-dianhydro-5-déoxy-D-glucitol-5-amino-2-nitrate

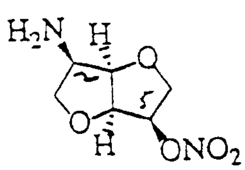
1,4:3,6-dianhydro-2-déoxy-D-manitol-2-amino-5-nitrate.

**3°)** Procédé de préparation d'un nitrate organique selon la revendication 1 ou la revendication 2, caractérisé en ce que l'on fait réagir :

I. soit un thioacide de type R-COOH, dans lequel R a la même signification que ci-dessus avec un dérivé de formule II : $(A)_n$-Y-B, dans laquelle A, Y, B et n ont la même signification que ci-dessus,

II. soit un dérivé de formule III : R-CO-(A)n, dans laquelle R, A et n ont la même signification que ci-dessus, avec un dérivé de formule Y-B, dans laquelle Y et B ont la même signification que ci-dessus, dans un solvant approprié et dans des conditions non épimérisantes.

**4°)** Procédé selon la revendication 3, caractérisé en ce que les dérivés de formule II sont choisis parmi :

| A |
|---|
| $C_6H_5\text{-}H_2C\text{-}OOC\text{-}HN\text{-}CH_2\text{-}CO$ |
| $(CH_3)_3C\text{-}OOC\text{-}HN\text{-}CH_2\text{-}CO$ |
| $HCl \,.\, H_2N\text{-}CH_2\text{-}CO$ |
| 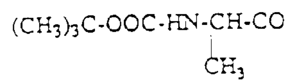 |
| 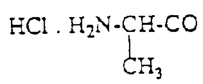 |
| 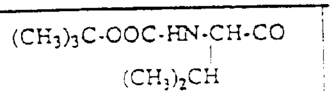 |
| 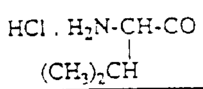 |
| 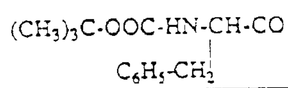 |
| 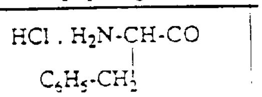 |
| 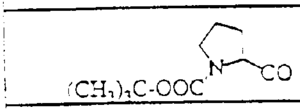 |
| 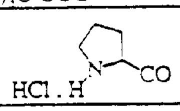 |

| Y-B | | | |
|---|---|---|---|
| 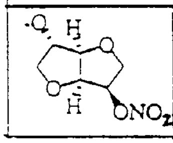 | 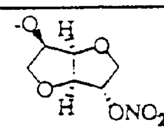 | 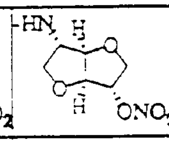 | 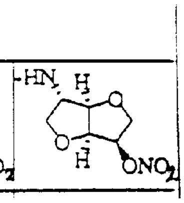 |

5°) Procédé selon la revendication 3, caractérisé en ce que le dérivé de formule III est :

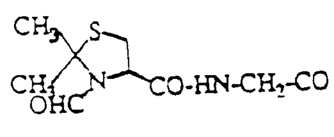

6°) Nitrate organique selon la revendication 1 ou la revendication 2, caractérisé en ce qu'il correspond au 2-O-[(3-t-butoxycarbonyl-4-L-thiazolidinyl) carbonyl]-, 5-O-nitro, 1,4:3,6-dianhydro-D-glucitol (18).

7°) Nitrate organique selon la revendication 1 ou la revendication 2, caractérisé en ce qu'il correspond au 2-O-[(4-L-thiazolidinyl)carbonyl]-, 5-O-nitro, 1,4:3,6-dianhydro-D-glucitol, monochlorhydrate (19).

8°) Nitrate organique selon la revendication 1 ou la revendication 2, caractérisé en ce qu'il correspond au L-cystéine, S-[(acétylamino)méthyl]-N-[(t-butoxy)carbonyl]-, ester du 5-O-nitro, 1,4:3,6-dianhydro-D-glucitol (20).

9°) Nitrate organique selon la revendication 1 ou la revendication 2, caractérisé en ce qu'il correspond au L-cystéine, S-[(acétylamino)méthyl]-, ester du 5-O-nitro, 1,4:3,6-dianhydro-D-glucitol, monochlorhydrate (21).

10°) Nitrate organique selon la revendication 1 ou la revendication 2, caractérisé en ce qu'il correspond au 2-O-[2-[(phénylméthyl)thio]benzoyl]-, 5-O-nitro, 1,4:3,6-dianhydro-D-glucitol (22).

**11°)** Nitrate organique selon la revendication 1 ou la revendication 2, caractérisé en ce qu'il correspond au 2-O-[2-[(acétylamino)méthyl]thio]benzoyl]-, 5-O-nitro, 1,4:3,6-dianhydro-D-glucitol (23).

**12°)** Nitrate organique selon la revendication 1 ou la revendication 2, caractérisé en ce qu'il correspond au 5-O-[(3-t-butoxycarbonyl-4-L-thiazolidinyl) carbonyl]-, 2-O-nitro, 1,4:3,6-dianhydro-D-glucitol (24).

**13°)** Nitrate organique selon la revendication 1 ou la revendication 2, caractérisé en ce qu'il correspond au 5-O-[(4-L-thiazolidinyl)carbonyl]-, 2-O-nitro, 1,4:3,6-dianhydro-D-glucitol, monochlorhydrate (25).

**14°)** Nitrate organique selon la revendication 1 ou la revendication 2, caractérisé en ce qu'il correspond au L-cystéine, S-[(acétylamino)méthyl]-N-[(t-butoxy)carbonyl]-, ester du 2-O-nitro, 1,4:3,6-dianhydro-D-glucitol (26).

**15°)** Nitrate organique selon la revendication 1 ou la revendication 2, caractérisé en ce qu'il correspond au L-cystéine, S-[(acétylamino)méthyl]-, ester du 2-O-nitro, 1,4:3,6-dianhydro-D-glucitol, monochlorhydrate (27).

**16°)** Nitrate organique selon la revendication 1 ou la revendication 2, caractérisé en ce qu'il correspond au 5-O-[2-[(phénylméthyl)thio]benzoyl]-, 2-O-nitro, 1,4:3,6-dianhydro-D-glucitol (28).

**17°)** Nitrate organique selon la revendication 1 ou la revendication 2, caractérisé en ce qu'il correspond au Bis[5-O[(2-thio)benzoyl]-, 2-O-nitro, 1,4:3,6-dianhydro-D-glucitol] (29).

**18°)** Nitrate organique selon la revendication 1 ou la revendication 2, caractérisé en ce qu'il correspond au 5-[[(4-L-thiazolidinyl)carbonyl]amino]-, 5-déoxy, 2-O-nitro, 1,4:3,6-dianhydro-L-iditol, monochlorhydrate (30).

**19°)** Nitrate organique selon la revendication 1 ou la revendication 2, caractérisé en ce qu'il correspond au 5-[[(3-formyl 2,2-diméthyl-4-L-thiazolidinyl)carbonyl]amino]-, 5-déoxy, 2-O-nitro, 1,4:3,6-dianhydro-L-iditol, monochlorhydrate (31).

**20°)** Nitrate organique selon la revendication 1 ou la revendication 2, caractérisé en ce qu'il correspond au 5-[[(2,2-diméthyl-4-L-thiazolidinyl)carbonyl] amino]-, 5-déoxy, 2-O-nitro, 1,4:3,6-dianhydro-L-iditol, monochlorhydrate (32).

**21°)** Nitrate organique selon la revendication 1 ou la revendication 2, caractérisé en ce qu'il correspond au L-cystéine, S-[(acétylamino)méthyl]-N-[(t-butoxy)carbonyl]-, amide du 5-amino, 5-déoxy, 2-O-nitro, 1,4:3,6-dianhydro-L-iditol (33).

**22°)** Nitrate organique selon la revendication 1 ou la revendication 2, caractérisé en ce qu'il correspond au L-cystéine, S-[(acétylamino)méthyl]-, amide du 5-amino, 5-déoxy, 2-O-nitro, 1,4:3,6-dianhydro-L-iditol, monochlorhydrate (34).

**23°)** Nitrate organique selon la revendication 1 ou la revendication 2, caractérisé en ce qu'il correspond au L-cystéine, S-[(2-nitro-phényl)thio]-N-[(t-nutoxy)carbonyl]-, amide du 5-amino, 5-déoxy, 2-O-nitro, 1,4:3,6-dianhydro-L-iditol (35).

**24°)** Nitrate organique selon la revendication 1 ou la revendication 2, caractérisé en ce qu'il correspond au L-méthionine, N-[(t-butoxy)carbonyl]-, amide du 5-amino, 5-déoxy, 2-O-nitro, 1,4:3,6-dianhydro-L-iditol (37).

**25°)** Nitrate organique selon la revendication 1 ou la revendication 2, caractérisé en ce qu'il correspond au 5-[[2-[(phénylméthyl)thio]benzoyl]amino]-, 5-déoxy, 2-O-nitro, 1,4:3,6-dianhydro-L-iditol (39).

**26°)** Nitrate organique selon la revendication 1 ou la revendication 2, caractérisé en ce qu'il correspond au 2-[[(4-L-thiazolidinyl)carbonyl]amino]-, 2-déoxy, 5-O-nitro, 1,4:3,6-dianhydro-D-glucitol, monochlorhydrate (40).

**27°)** Nitrate organique selon la revendication 1 ou la revendication 2, caractérisé en ce qu'il correspond au L-cystéine, S-[(acétylamino)méthyl]-N-[(t-butoxy)carbonyl]-, amide du 2-amino, 2-déoxy, 5-O-nitro, 1,4:3,6-dianhydro-D-glucitol (41).

**28°)** Nitrate organique selon la revendication 1 ou la revendication 2, caractérisé en ce qu'il correspond au L-cystéine, S-[(acétylamino)méthyl]-, amide du 2-amino, 2-déoxy, 5-O-nitro, 1,4:3,6-dianhydro-D-glucitol, monochlorhydrate (42).

**29°)** Nitrate organique selon la revendication 1 ou la revendication 2, caractérisé en ce qu'il correspond au L-méthionine, N-[(t-butoxy)carbonyl]-, amide du 2-amino, 2-déoxy, 5-O-nitro, 1,4:3,6-dianhydro-D-glucitol (43).

**30°)** Nitrate organique selon la revendication 1 ou la revendication 2, caractérisé en ce qu'il correspond au 2-[[2-[(phénylméthyl)thio]benzoyl]amino]-, 2-déoxy, 5-O-nitro, 1,4:3,6-dianhydro-D-glucitol (45).

**31°)** Nitrate organique selon la revendication 1 ou la revendication 2, caractérisé en ce qu'il correspond au 5-[[2-[[(4-L-thiazolidinyl)carbonyl]amino]1-oxo éthyl] amino], 5-déoxy, 2-O-nitro, 1,4:3,6-dianhydro-L-iditol, monochlorhydrate (46).

**32°)** Nitrate organique selon la revendication 1 ou la revendication 2, caractérisé en ce qu'il correspond au 5-[[[[2-[(phénylméthyl)thio] benzoyl] amino]1-oxo éthyl]amino]-, 5-déoxy, 2-O-nitro, 1,4:3,6-dianhydro-L-iditol (47).

**33°)** Nitrate organique selon la revendication 1 ou la revendication 2, caractérisé en ce qu'il correspond au 5-[[2-[[(3-fomyl-2,2-diméthyl-4-L- thiazolidinyl)carbonyl]amino]1-oxo éthyl]amino]-, 5-déoxy, 2-O-nitro, 1,4:3,6-dianhydro-L-iditol (48).

**34°)** Glycine, N-[(t-butoxy)carbonyl]-, ester du 5-O-nitro, 1,4:3,6-dianhydro-D-glucitol (1), produit intermédiaire du procédé selon la revendication 3.

**35°)** Glycine, ester du 5-O-nitro, 1,4:3,6-dianhydro-D-glucitol, monochlorhydrate (2), produit intermédiaire du procédé selon la revendication 3.

**36°)** Glycine, ester du 2-O-nitro, 1,4:3,6-dianhydro-D-glucitol, monochlorhydrate (3), produit intermédiaire du procédé selon la revendication 3.

**37°)** Glycine, N-[(benzyloxy)carbonyl]-, amide du 5-amino, 5-déoxy, 2-O-nitro, 1,4:3,6-dianhydro-L-iditol (4), produit intermédiaire du procédé selon la revendication 3.

**38°)** Glycine, N-[(t-butoxy)carbonyl]-, amide du 5-amino, 5-déoxy, 2-O-nitro, 1,4:3,6-dianhydro-L-iditol (5), produit intermédiaire du procédé selon la revendication 3.

**39°)** Glycine, amide du 5-amino, 5-déoxy, 2-O-nitro, 1,4:3,6-dianhydro-L-iditol, monochlorhydrate (6), produit intermédiaire du procédé selon la revendication 3.

**40°)** L-alanine, N-[(t-butoxy)carbonyl]-, amide du 5-amino, 5-déoxy, 2-O-nitro, 1,4:3,6-dianhydro-L-iditol (7), produit intermédiaire du procédé selon la revendication 3.

**41°)** L-alanine, amide du 5-amino, 5-déoxy, 2-O-nitro, 1,4:3,6-dianhydro-L-iditol (8), produit intermédiaire du procédé selon la revendication 3.

**42°)** L-valine, N-[(t-butoxy)carbonyl]-, amide du 5-amino, 5-déoxy, 2-O-nitro, 1,4:3,6-dianhydro-L-iditol (9), produit intermédiaire du procédé selon la revendication 3.

**43°)** L-phénylalanine, N-[(t-butoxy)carbonyl]-, amide du 5- amino, 5-déoxy, 2-O-nitro-, 1,4:3,6-dianhydro-L-iditol (11), produit intermédiaire du procédé selon la revendication 3.

**44°)** L-phénylalanine, N-[(t-butoxy)carbonyl]-, amide du 5- amino, 5-déoxy, 2-O-nitro, 1,4:3,6-dianhydro-L-iditol, monochlorhydrate (12), produit intermédiaire du procédé selon la revendication 3.

**45°)** L-proline, N-[(t-butoxy)carbonyl]-, amide du 5-amino, 5-déoxy, 2-O-nitro, 1,4:3,6-dianhydro-L-iditol (13), produit intermédiaire du procédé selon la revendication 3.

**46°)** L-proline, amide du 5-amino, 5-déoxy, 2-O-nitro, 1,4:3,6-dianhydro-L-iditol (14), produit intermédiaire du procédé selon la revendication 3.

**47°)** Glycine, N-[(t-butoxy)carbonyl]-, amide du 2-amino, 2-déoxy, 5-O-nitro, 1,4:3,6-dianhydro-D-glucitol (15), produit intermédiaire du procédé selon la revendication 3.

**48°)** Glycine, amide du 2-amino, 2-déoxy, 5-O-nitro, 1,4:3,6-dianhydro-D-glucitol (16), produit intermédiaire du procédé selon la revendication 3.

**49°)** Médicament, caractérisé en ce qu'il comprend au moins un nitrate organique selon la revendication 1, la revendication 2 ou l'une quelconque des revendications 6 à 33, seul ou en association avec un ou plusieurs principes actifs et/ou adjuvants pharmaceutiquement compatibles.

**50°)** Médicament caractérisé en ce qu'il comprend un dérivé selon l'une quelconque des revendications 34 à 48.

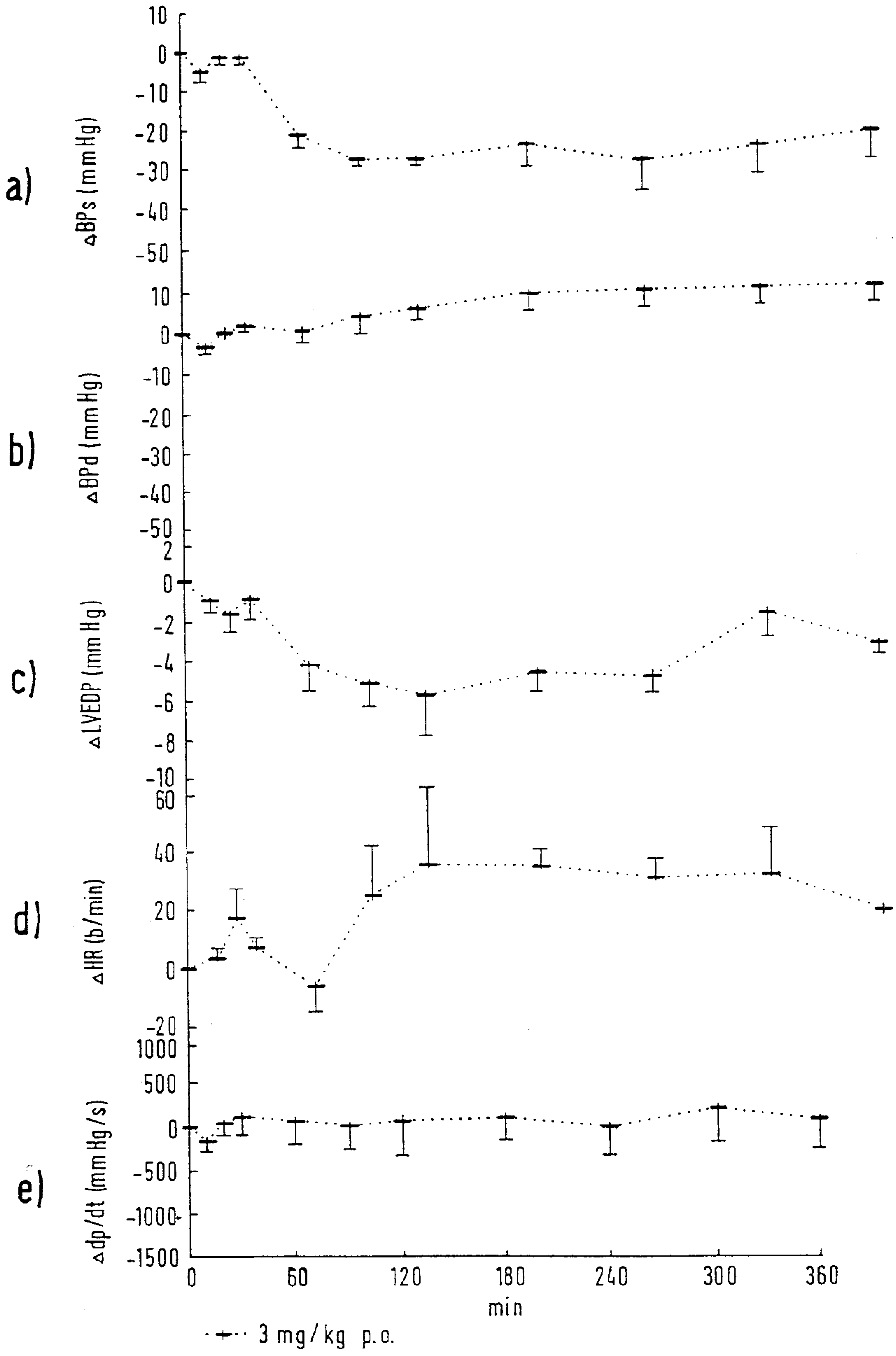
a)
ΔBPs (mmHg)
10
0
-10
-20
-30
-40
-50
b)
ΔBPd (mm Hg)
10
0
-10
-20
-30
-40
-50
c)
ΔLVEDP (mm Hg)
2
0
-2
-4
-6
-8
-10
d)
ΔHR (b/min)
60
40
20
0
-20
e)
Δdp/dt (mm Hg/s)
1000
500
0
-500
-1000
-1500
0
60
120
180
240
300
360
min
3 mg/kg p.o.

Fig. 1

a)

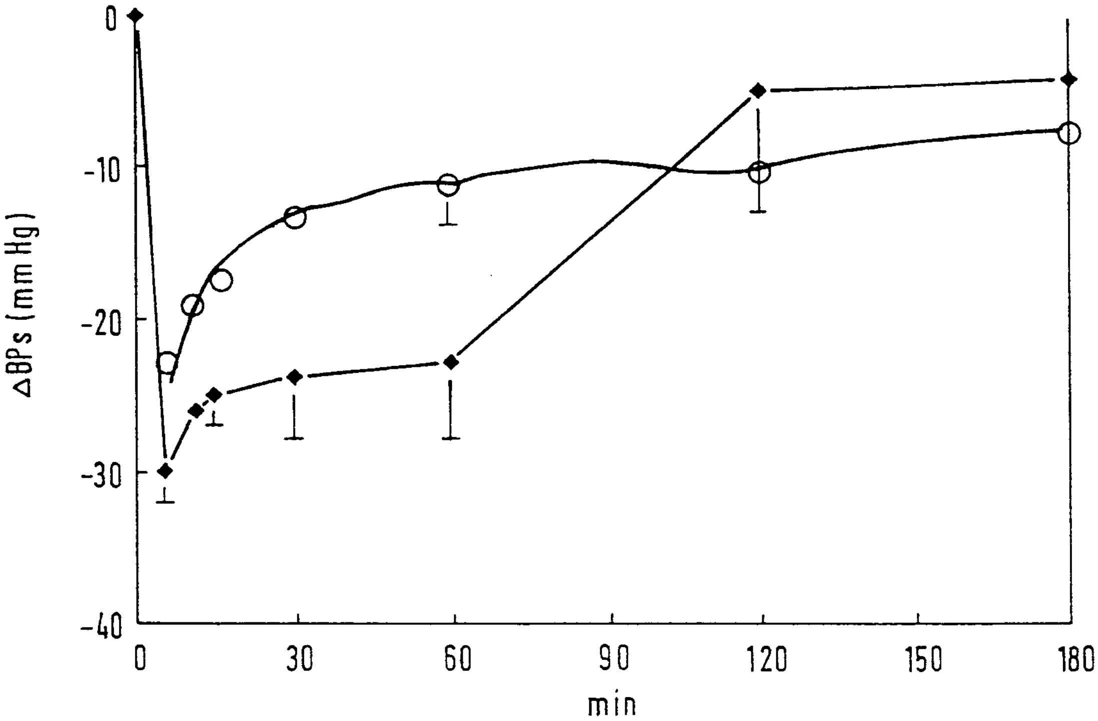
0
-10
-20
-30
-40
ΔBPs (mm Hg)
0
30
60
90
120
150
180
min

b)

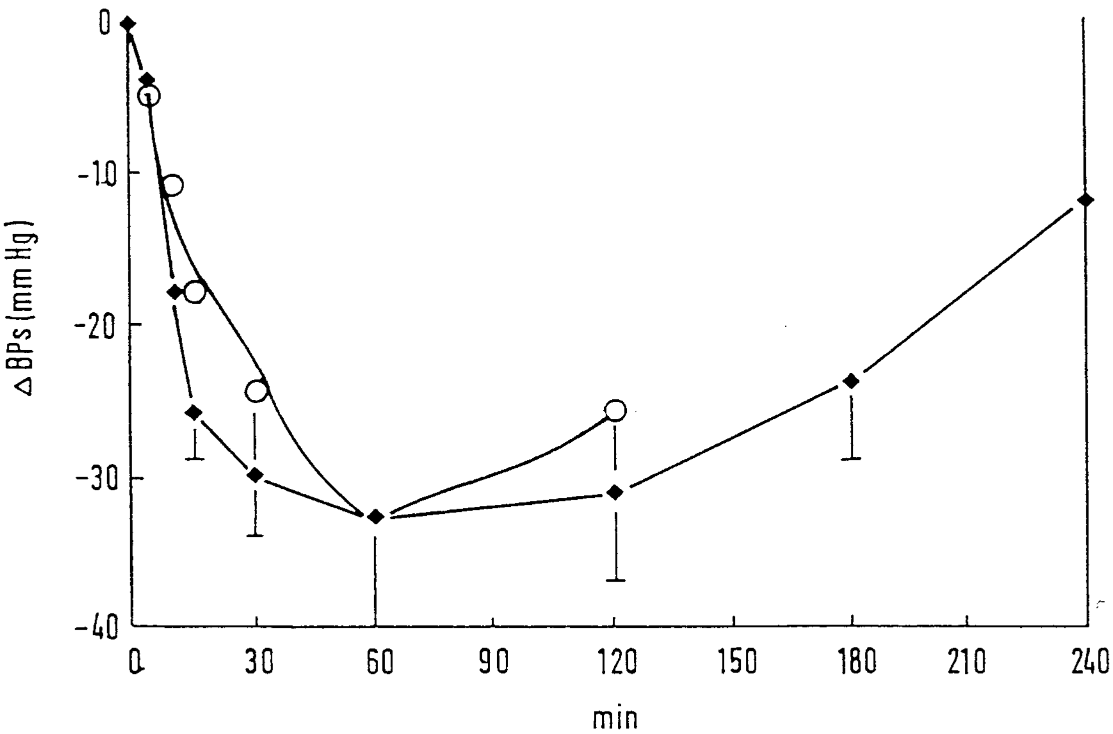
0
-10
-20
-30
-40
ΔBPs (mm Hg)
0
30
60
90
120
150
180
210
240
min

Fig. 2

Office européen des brevets

# RAPPORT PARTIEL DE RECHERCHE EUROPEENNE

qui selon la règle 45 de la Convention sur le brevet européen est consideré, aux fins de la procédure ultérieure comme le rapport de la recherche européenne

Numero de la demande: EP 92 20 2500

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 5) |
|---|---|---|---|
| A | EP-A-0 290 885 (CHIESI FARMACEUTICI)<br>* revendications 1,12 * | 1,49,50 | C07D493/04<br>C07D277/06<br>C07K5/06<br>A61K31/425<br>A61K31/34<br>//(C07D493/04, 307:00,307:00) |
| A | EP-A-0 114 270 (HEINRICH MACK NACHF.)<br>* revendications 1,10,11; abrégé * | 1,49,50 | |
| D,A | EP-A-0 362 575 (SCHWARZ PHARMA)<br>* revendications 1,8; abrégé * | 1,49,50 | |
| P,X | WO-A-9 204 337 (CEDONA PHARMACEUTICALS)<br>* revendications 1,5,6 * | 1,49,50 | |

**DOMAINES TECHNIQUES RECHERCHES (Int. Cl. 5)**

C07D
C07K
A61K

## RECHERCHE INCOMPLETE

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.
Revendications ayant fait l'objet de recherches complètes:
Revendications ayant fait l'objet de recherches incomplètes:
Revendications n'ayant pas fait l'objet de recherches:
Raison pour la limitation de la recherche:

voir feuille supplémentaire C

| Lieu de la recherche | Date d'achèvement de la recherche | Examinateur |
|---|---|---|
| LA HAYE | 30 SEPTEMBRE 1992 | VOYIAZOGLOU D. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04E08)

EP 92 20 2500

-C-

Le domaine des revendications est si grande qu'ils ne satisfaient pas Article 84 EPC et qu'une recherche economique n'est guère possible (Directives B III 2ii) (revendications doivent être claires et concises). La recherche était limitée aux composés qui sont dans la description.

Revendications ayant fait l'objet de recherches complètes: 6-48,50

Revendications ayant fait l'objet de recherches incomplètes: 1-5,49